# EUROPÄISCHE PATENTSCHRIFT

(11) **EP 2 432 405 B1**
(45) Veröffentlichungstag und Bekanntmachung des Hinweises auf die Patenterteilung: **30.11.2016**
(21) Anmeldenummer: 09796635.2
(22) Anmeldetag: 23.12.2009
(51) Int. Cl.: A61B 17/221, A61B 17/00

(54) **WECHSELGRIFFSYSTEM UND MEDIZINISCHES INSTRUMENT**
CHANGE-OUT HANDLE SYSTEM AND MEDICAL INSTRUMENT
SYSTÈME DE PRÉHENSION INTERCHANGEABLE ET INSTRUMENT MÉDICAL

(30) Priorität: 22.05.2009 DE 102009022379
(43) Veröffentlichungstag der Anmeldung: 28.03.2012
(73) Patentinhaber: Urotech Medizinische Technologie GmbH, 83101 Achenmühle (DE)
(72) Erfinder: UIHLEIN, Bernhard, 72581 Dettingen (DE); PINKOWSKI, Wolfhard, 85609 Aschheim (DE); MBARGA, Dieudonné, 83101 Achenmühle (DE); SCHWARZ, Werner, 83324 Ruhpolding (DE)
(74) Vertreter: Patentanwälte Ruff, Wilhelm, Beier, Dauster & Partner mbB
(86) Internationale Anmeldenummer: PCT/EP2009/009256
(87) Internationale Veröffentlichungsnummer: WO 2010/133245

(56) Entgegenhaltungen:
- EP-A1- 0 943 293
- US-A- 2 113 246
- US-A- 5 779 686
- US-A- 6 007 560
- US-A- 6 053 934
- US-A1- 2002 010 459
- US-A1- 2002 026 202
- US-A1- 2005 113 862
- US-A1- 2005 182 292
- US-A1- 2008 132 940

## Beschreibung

Die Erfindung bezieht sich auf ein Wechselgriffsystem zum lösbaren Ankoppeln zweier langgestreckter, axial relativbeweglicher Funktionsteile und auf ein entsprechendes medizinisches Instrument.

Im Bereich der Medizintechnik gibt es verschiedentlich medizinische Instrumente, die zwei langgestreckte, axial relativbewegliche Funktionsteile beinhalten, insbesondere für Endoskopieanwendungen. Wichtige Beispiele sind unter anderem Steinfangkorbinstrumente mit entfaltbarem Drahtkorb zum Einfangen von Steinen und dergleichen in Gewebehohlräumen, Drahtfilterinstrumente und Führungsdrahteinheiten für Katheterinstrumente. Bei den beiden langgestreckten, axial relativbeweglichen Funktionsteilen kann es sich z.B. um einen sogenannten Zugdraht und einen diesen umgebenden Schlauch, d.h. Umhüllung, handeln.

Durch die axiale Relativbewegung der beiden Funktionsteile wird ein distales Funktionselement in wenigstens zwei unterschiedliche Funktionszustände gesteuert, im Fall eines Steinfangkorbinstruments z.B. ein distaler Drahtkorb wahlweise in einen in den Schlauch eingezogenen, zusammengefalteten Zustand oder einen aus dem Schlauch vorgeschobenen, aufgefalteten Zustand. Am proximalen Ende werden die beiden Funktionsteile typischerweise an einen Bediengriff derart angekoppelt, dass der Schlauch mit einem stationären Griffabschnitt und der Zugdraht mit einem aktiv zu betätigenden Griffabschnitt verbunden ist. Bei derartigen herkömmlichen Anordnungen bleibt folglich der Schlauch nach dem Einführen des Instruments in einen Körpergewebekanal gegenüber diesem stationär, während der Zugdraht durch die proximale Benutzerbetätigung axial vor und zurück bewegt wird, um beispielsweise einen Steinfangkorb aus dem Schlauch distal herauszubewegen und aufzufalten und ihn nach Einfangen eines Steins wieder bis zum Festhalten desselben zurückzubewegen. Der Drahtkorb verändert somit während dieser Funktionsbewegung seine axiale Position im Gewebekanal, was unerwünscht sein kann bzw. die Steineinfangfunktion erschweren kann. Herkömmliche Griffsysteme für solche Anwendungen haben außerdem häufig das Problem, dass ein Lösen der Funktionsteile vom Griff oftmals nicht oder nur schwer bzw. nur unter Verwendung loser, verliergefährdeter Verbindungsteile und/oder spezieller Werkzeuge möglich ist.

Die Offenlegungsschrift US 2005/0113862 A1 offenbart ein Griffsystem zum lösbaren Ankoppeln zweier langgestreckter, axiale relativbeweglicher Funktionsteile eines medizinischen Instruments, insbesondere einer Führungsdrahteinheit, in Form eines mittigen Zugdrahts und eines diesen umgebenden Schlauchs, der zwischen seinem distalen und seinem proximalen Ende einen verformbaren Abschnitt und zwischen diesem verformbaren Abschnitt und dem proximalen Ende einen elastisch vorspannenden Abschnitt aufweist, durch dessen Vorspannkraft sich die axiale Länge des verformbaren Abschnitts ändern lässt. Der Zugdraht und der Schlauch sind am distalen und am proximalen Ende jeweils fest miteinander verbunden. Zur Betätigung kann ein Griff mit einem vorderen und einem hinteren Griffteil verwendet werden, wobei der vordere Griffteil eine Durchführungsöffnung aufweist, durch die der Zugdraht und der ihn umgebende Schlauch mit einem proximalen Endabschnitt hindurchgeführt sind, der den elastisch vorspannenden Abschnitt umfasst. Dabei ist der proximale Endabschnitt von Zugdraht und Schlauch lose in einer axialen Aufnahmebohrung des hinteren Griffteils aufgenommen, und der sich distal an den elastisch vorspannenden Abschnitt anschließende Schlauchabschnitt ist lösbar mittels einer Schraubklemmung am vorderen Griffteil fixiert. Die beiden Griffteile sind um eine vorgebbare Länge axial relativbeweglich aneinander geführt. Durch die Wirkung des elastisch vorspannenden Abschnitts wird der verformbare Schlauchabschnitt in einem ersten Formzustand gehalten. Durch Betätigen des hinteren Griffteils wird der verformbare Schlauchabschnitt aus diesem ersten in einen zweiten Formzustand gebracht. Dabei dient der erste Formzustand zur Verankerung der Führungsdrahteinheit in einem Gewebekanal, um beispielsweise ein Katheterrohr über die Führungsdrahteinheit aufschieben und einführen zu können, während der zweite Formzustand einen Einführzustand darstellt, in welchem die Führungsdrahteinheit in einen Gewebekanal bzw. aus diesem wieder herausbewegt werden kann.

Weitere herkömmliche medizinische Instrumente der eingangs genannten Art mit Wechselgriffsystem sind in den Patentschriften US 5,779,686, gemäß dem Oberbegriff des Anspruchs 1,

US 2,113,246 und US 6,053,934 sowie in der Offenlegungsschrift US 2002/0026202 A1 offenbart.

Der Erfindung liegt als technisches Problem die Bereitstellung eines Wechselgriffsystems der eingangs genannten Art, das ein vergleichsweise einfaches und funktionssicheres, lösbares Koppeln der Funktionsteile mit den Griffteilen ermöglicht, und eines zugehörigen medizinischen Instruments zugrunde.

Die Erfindung löst dieses Problem durch die Bereitstellung eines Wechselgriffsystems mit den Merkmalen des Anspruchs 1 und eines medizinischen Instruments mit den Merkmalen des Anspruchs 10.

Das Wechselgriffsystem nach Anspruch 1 weist einen ersten und einen zweiten Griffteil auf, die axial relativbeweglich hintereinander angeordnet sind, wobei ein erstes Kopplungsmittel zum lösbaren, axial bewegungsstarren Koppeln eines ersten der beiden Funktionsteile mit dem ersten Griffteil und ein zweites Kopplungsmittel zum lösbaren, axial bewegungsstarren Koppeln des zweiten der beiden Funktionsteile mit dem zweiten Griffteil vorgesehen sind. Dabei weist der zweite Griffteil eine Durchführungsöffnung zum Durchführen wenigstens des ersten der beiden Funktionsteile auf. Diese konstruktiven Maßnahmen schaffen die Voraussetzung für ein sehr einfaches, lösbares Ankoppeln der Funktionsteile an die Griffteile, ohne dass dazu zwingend weitere, z.B. lose Teile oder Werkzeuge benötigt werden. Indem das Koppeln sowohl des ersten Funktionsteils mit dem ersten Griffteil als auch des zweiten Funktionsteils mit dem zweiten Griffteil axial bewegungsstarr realisiert ist, ist gewährleistet, dass jedes der beiden Funktionsteile einer Axialbewegung des zugehörigen Griffteils exakt und praktisch spielfrei folgen kann. Erfindungsgemäß beinhaltet der erste Griffteil einen Griffkörper aus einer unteren Griffschale und einer auf diese aufklipsbaren oberen Griffschale, und der zweite Griffteil weist ein an der Oberseite der oberen Griffschale herausragendes, daumenbetätigtes Pilzkopfteil auf. Dies ermöglicht eine besonders komfortable und funktionssichere Bedienung der Funktionsteile.

In Weiterbildung der Erfindung weist das erste und/oder das zweite Kopplungsmittel ein zugeordnetes betätigbares Sicherungsmittel auf, mit dem die jeweils zugehörige Koppelverbindung von Funktionsteil und Griffteil gesichert wird. Dies schützt weitergehend vor einem etwaigen unbeabsichtigten Lösen der betreffenden Koppelverbindung.

In Weiterbildung der Erfindung weist das erste Kopplungsmittel ein erstes Koppelelement am ersten Griffteil zum lösbaren Koppeln mit einem ersten Gegenkoppelelement an einem hinteren Endbereich des ersten Funktionsteils auf. Damit kann durch die beiden zusammenwirkenden Elemente das erste Funktionsteil mit seinem hinteren Endbereich lösbar an den ersten Griffteil gekoppelt werden.

In Weiterbildung der Erfindung weist das zweite Kopplungsmittel ein zweites Koppelelement zum lösbaren Koppeln mit einem zweiten Gegenkoppelelement an einem hinteren Endbereich des zweiten Funktionsteils auf. Mit diesen beiden Elementen kann folglich das zweite Funktionsteil mit seinem hinteren Endbereich lösbar an den zweiten Griffteil gekoppelt werden.

In Weiterbildung der Erfindung beinhaltet das erste und/oder das zweite Kopplungsmittel eine jeweilige Nut oder Nutaufnahme und eine jeweils korrespondierende Nutaufnahme bzw. Nut als formschlüssiges Koppelelement-Gegenkoppelelement-Paar oder eine jeweilige Schlitzöffnung zur Bildung eines formschlüssigen Paares mit einem jeweiligen Haltering- oder Wulstelement. Der Formschluss dieser Kopplung gewährleistet in einer vorteilhaften Weise das lösbare und axial bewegungsstarre Koppeln von jeweiligem Funktionsteil und Griffteil. In weiterer Ausgestaltung der Erfindung ist für das jeweilige Kopplungspaar ein betätigbares Verriegelungselement als Sicherungsmittel vorgesehen, mit welchem die Verbindung gegen Lösen verriegelt und zum Lösen entriegelt wird. Dies sichert in vorteilhafter Weise die jeweilige Verbindung gegen unbeabsichtigtes Lösen.

In Weiterbildung der Erfindung ist ein variables Hubbegrenzungselement vorgesehen, mit dem sich der relative axiale Hubbewegungsweg zwischen erstem und zweitem Griffteil variabel einstellen lässt. Dies ist z.B. im Fall von Steinfangkorbinstrumenten nützlich, wenn die Griffteile für Instrumente mit verschiedenen Drahtkorblängen verwendet werden.

In Weiterbildung der Erfindung ist ein elastisches Vorspannelement vorgesehen, das in Axialrichtung zwischen den beiden Griffteilen wirkt. Dadurch lassen sich die beiden Griffteile vorgespannt in einer ihrer beiden axialen Endstellungen halten, aus der heraus sie dann vom Benutzer aktiv in Richtung der anderen Endstellung relativbewegt werden können.

In Weiterbildung der Erfindung bildet der zweite Griffteil einen aktiv zu betätigenden Griffteil, der vom Benutzer aktiv betätigt wird, um die beabsichtigte Nutzfunktion der beiden Funktionsteile auszuführen.

In einer weiteren vorteilhaften Weiterbildung der Erfindung ist der erste Griffteil als ein Griffkörper mit einer Schlittenführung ausgestaltet, und der zweite Griffteil bildet ein aktiv zu betätigendes Schieberelement, das durch die Schlittenführung axial beweglich am Griffkörper geführt ist. Die Funktionsbewegung zum Ausführen der beabsichtigten Nutzfunktion der beiden ankoppelbaren Funktionsteile beinhaltet in diesem Fall folglich ein aktives Bewegen des Schieberelements und somit des an dieses gekoppelten Funktionsteils, während der Griffkörper und der mit ihm gekoppelte Funktionsteil im Wesentlichen stationär bleiben können.

In Ausgestaltung dieses Erfindungsaspekts beinhaltet das Schieberelement einen Schlittenkörper und einen daran querbeweglich gehaltenen Bedienkopfkörper, der das zugeordnete Sicherungsmittel zum Sichern der Koppelverbindung von Griffbedienteil und zweitem Funktionsteil bildet, wobei er zwischen einer verrastenden Schiebebedienstellung und einer entsichernden Stellung querbeweglich ist. Der Bedienkopfkörper erfüllt auf diese Weise sowohl eine Bedienelementfunktion im normalen Gebrauch als auch eine Kopplungsfunktion zum lösbaren Halten des zugehörigen Funktionsteils.

Das medizinische Instrument nach Anspruch 10 beinhaltet ein erfindungsgemäßes Wechselgriffsystem in Kombination mit einer speziellen medizinischen Instrumenteneinheit.

In einer Weiterbildung der Erfindung handelt es sich um ein Endoskopieinstrument, wobei die medizinische Instrumenteneinheit mittels einer Teleskop-Adapterhülse an einen Endoskopiegriffkörper ankoppelbar ist. Mit dieser teleskopisch in ihrer Länge veränderbaren Adapterhülse kann die medizinische Instrumenteneinheit in ihrem an das Wechselgriffsystem angekoppelten Gebrauchszustand sehr bequem an den Endoskopieteil angekoppelt werden.

Die medizinische Instrumenteneinheit ist in Weiterbildung der Erfindung in spezieller Weise derart ausgestaltet, dass sie vergleichsweise einfach, zuverlässig und funktionssicher mit dem erfindungsgemäßen Wechselgriffsystem gekoppelt werden kann. Dazu sind die beiden langgestreckten Funktionsteile, die einen wesentlichen Teil der Instrumenteneinheit bilden, mit geeigneten Gegenkoppelelementen versehen.

In weiterer Ausgestaltung der Erfindung sind das erste Gegenkoppelelement durch ein am ersten Funktionsteil vorgesehenes erstes Anschlussstück und das zweite Gegenkoppelelement durch ein am zweiten Funktionsteil vorgesehenes zweites Anschlussstück gebildet, wobei in Axialrichtung zwischen den beiden Anschlussstücken ein elastisches Element wirkt. Dies kann insbesondere dazu dienen, die beiden Funktionsteile durch die Wirkung des elastischen Elementes in eine vorgebbare axiale Relativposition vorzuspannen, aus der heraus sie durch aktive Benutzerbetätigung axial gegeneinander bewegt werden können.

In Weiterbildung dieses Erfindungsaspekts sind die beiden Funktionsteile durch einen Zugdraht und einen diesen umgebenden Schlauch z.B. eines medizinischen Endoskopieinstruments gebildet. In weiterer Ausgestaltung der Erfindung stellt hierbei der Zugdraht das erste Funktionsteil und der Schlauch das zweite Funktionsteil dar. In einer Realisierung, bei welcher der vordere Griffteil bzw. das Schieberelement den aktiv zu betätigenden Griffteil bildet, hat dies zur Folge, dass der Schlauch das aktiv bewegte Funktionsteil bildet, während der Zugdraht das demgegenüber stationär verbleibende Funktionsteil bildet. Dies hat den in vielen Fällen sehr erwünschten Vorteil, dass der Zugdraht und ein an ihm ggf. vorgesehenes distales Funktionselement während der Funktionsbetätigung axial im Wesentlichen ortsfest bleibt, z.B. in seiner in einen Gewebekanal eingeführten Lage, und im Wesentlichen nur der Schlauch relativ zum Gewebekanal axial bewegt wird.

In weiterer Ausgestaltung dieses Erfindungsaspekts ist die medizinische Instrumenteneinheit ein Steinfangkorbinstrument. Für dieses haben die vorstehend genannten Ausführungsvarianten mit aktiv betätigtem vorderem Griffteil bzw. aktiv betätigtem Schieberelement den Vorteil, dass sich die axiale Lage des Steinfangkorbs im Gewebekanal während der Auffaltbewegung und der Zusammenziehbewegung weitestgehend konstant halten lässt. Dies erleichtert beträchtlich das funktionssichere und zuverlässige Einfangen und Festhalten eines Steins oder dergleichen.

In noch einer weiteren Ausgestaltung dieses Erfindungsaspekts ist eine den Schlauch umgebende Einführhilfshülse vorgesehen, die in einer Ruheposition lösbar am vorderen Griffteil angebracht ist und aus der Ruheposition nach vorn bis zum distalen Endbereich von Zugdraht und Schlauch in eine Einführhilfsposition bewegbar ist. In der Einführhilfsposition erleichtert die Einführhilfshülse das Einführen des distalen Endbereichs von Zugdraht und Schlauch und schützt dadurch im Fall eines Steinfangkorbinstruments auch das empfindliche Steinfangkörbchen.

Vorteilhafte Ausführungsformen der Erfindung sind in den Zeichnungen dargestellt und werden nachfolgend beschrieben. Hierbei zeigen:
- Fig. 1A: eine Seitenansicht eines medizinischen Steinfangkorbinstruments in eingezogenem Korbzustand,
- Fig. 1 B: eine Seitenansicht eines proximalen Anschlussendabschnitts des Steinfangkorbinstruments von Fig. 1A,
- Fig. 1C: eine Längsschnittansicht des Anschlussendabschnitts von Fig. 1B,
- Fig. 2A bis 2C: Ansichten entsprechend den Fig. 1A bis 1C im Zustand mit voll entfaltetem Steinfangkorb,
- Fig. 3A bis 3C: Ansichten entsprechend den Figuren 1A bis 1C in einem Zustand mit eingefangenem und festgehaltenem Stein,
- Fig. 4A: eine Explosions-Längsschnittansicht eines Wechselgriffsystems mit einer Griffeinheit für das Steinfangkorbinstrument der Fig. 1A bis 3C,
- Fig. 4B und 4C: eine Längsschnittansicht bzw. Seitenansicht der Griffeinheit im montierten Zustand mit einem Griffüberrohr in einer vorderen Endstellung,
- Fig. 4D: eine Seitenansicht entsprechend Fig. 4C, jedoch mit dem Griffüberrohr in einer hinteren Endstellung,
- Fig. 4E: eine Seitenansicht entsprechend Fig. 4C, jedoch in einem Zustand mit auseinandergezogenen Griffteilen,
- Fig. 5A: eine Seitenansicht entsprechend Fig. 4D mit eingefügtem Steinfangkorbinstrument vor einem endgültigen Ankoppeln,
- Fig. 5B: eine Seitenansicht entsprechend Fig. 5A mit fertig an die Griffeinheit angekoppeltem Steinfangkorbinstrument,
- Fig. 6A und 6B: Querschnittansichten längs der Linie VIa-VIa von Fig. 5A bzw. längs der Linie VIb-VIb von Fig. 5B,
- Fig. 7: eine Draufsicht auf ein Koppelelementteil einer in den Fig. 6A und 6B veranschaulichten Koppelverbindung,
- Fig. 8: eine Draufsicht auf ein Sicherungselement für die in den Fig. 6A und 6B veranschaulichte Koppelverbindung,
- Fig. 9A und 9B: jeweils eine Seitenansicht entsprechend Fig. 5B im Bedienzustand mit ausgefahrenem bzw. eingezogenen Steinfangkorb,
- Fig. 10A bis 10C: positionsvergleichende Seitenansichten des Steinfangkorbinstruments ohne Griffeinheit im Zustand mit eingezogenem Fangkorb, herausbewegtem Fangkorb bzw. im Zustand mit eingefangenem und festgehaltenem Stein,
- Fig. 11A und 11B: eine Seitenansicht bzw. eine Draufsicht auf den vorderen Endbereich des vorderen Griffteils für eine Variante mit einem Schieberelement als Bedienbetätigungsmittel statt einer Griffmulde,
- Fig. 12A: eine Seitenansicht des vorderen Griffteils für eine Variante mit ankoppelbarer Einführhilfshülse,
- Fig. 12B: eine Seitenansicht der an das Griffteil gemäß Fig. 12A ankoppelbaren Einführhilfshülse,
- Fig. 12C: die Ansicht von Fig. 12A mit angekoppelter Einführhilfshülse,
- Fig. 13A und 13B: Seitenansichten entsprechend den Fig. 4E bzw. 4C für eine Griffvariante mit elastischem Vorspannelement für die beiden Griffteile in ausgezogener bzw. zusammengedrückter Griffendstellung,
- Fig. 14: eine Ansicht entsprechend Fig. 13B für eine Griffvariante mit variabler Hubbegrenzung,
- Fig. 15: eine Explosionsansicht eines weiteren, z.B. für medizinische Steinfangkorbinstrumente geeigneten Wechselgriffsystems,
- Fig. 16: eine Seitenansicht des Wechselgriffsystems von Fig. 15 im zusammengebauten Zustand bei abgenommener Oberschale,
- Fig. 17: eine Seitenansicht des zusammengebauten Wechselgriffsystems von Fig. 16 von schräg unten,
- Fig. 18A bis 18C: eine Seitenansicht, Vorderansicht bzw. Rückansicht eines Bedienkopfkörpers des Wechselgriffsystem von Fig. 15,
- Figur 19A und 19B: Seitenansichten eines zum Ankoppeln an das Wechselgriffsystem von Fig. 15 gestalteten Endbereichs einer medizinischen Instrumenteneinheit in einem vollständig zusammengeschobenen bzw. etwas auseinanderbewegten Zustand ihrer beiden langgestreckten Funktionsteile,
- Fig. 19C und 19: eine jeweilige Längsschnittansicht zu den Ansichten von Fig. 19A bzw. 19B,
- Fig. 20A und 20B: Seitenansichten eines medizinischen Steinfangkorbinstruments mit längsgeschnittenem Wechselgriffsystem gemäß Fig. 15 im Zustand mit eingezogenem bzw. entfaltetem Körbchen und
- Fig. 21: eine Perspektivansicht eines griffseitigen Teils eines Endoskops mit an einen Endoskopiegriffkörper angekoppeltem medizinischen Instrument entsprechend den Fig. 20A und 20B.

In den Figuren ist die Erfindung exemplarisch in Anwendungen von Wechselgriffsystemen für medizinische Steinfangkorbinstrumente veranschaulicht, der Fachmann erkennt daraus jedoch ohne Weiteres, dass sich das erfindungsgemäße Wechselgriffsystem in gleicher Weise für jegliche andere Anwendungen und insbesondere medizinischen Instrumente eignet, bei denen zwei langgestreckte, axial relativbewegliche Funktionsteile lösbar an ein Griffsystem anzukoppeln sind, typischerweise zu dem Zweck, die Funktionsteile über das Griffsystem zu bedienen.

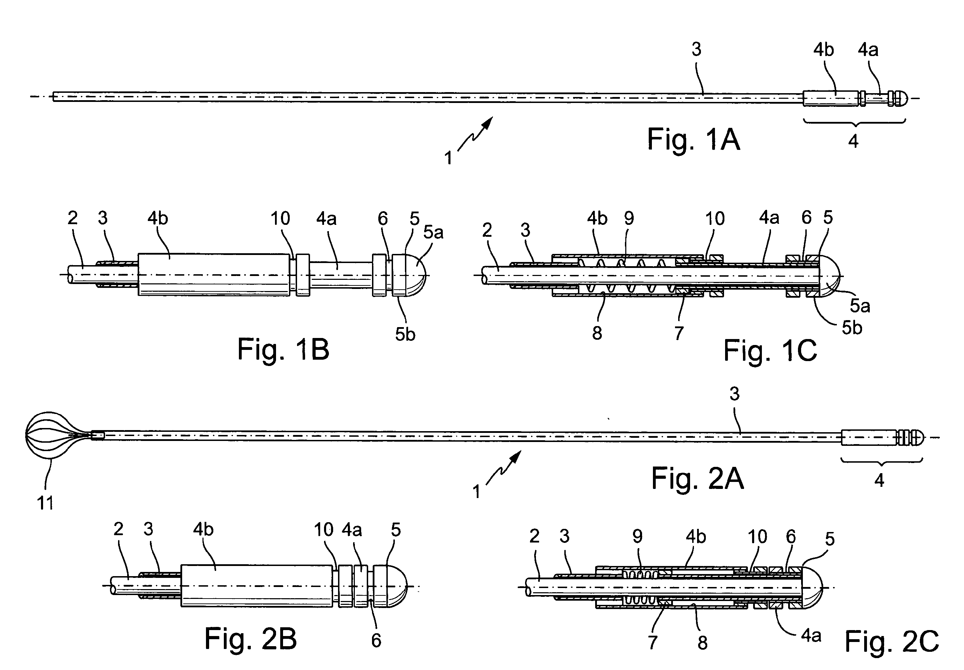

In den Fig. 1A bis 1C ist ein Steinfangkorbinstrument 1 dargestellt, das wie üblich einen langgestreckten, mittigen Draht 2, üblicherweise Zugdraht genannt, und eine diesen umgebende Umhüllung 3 beinhaltet, vorliegend auch als Schlauch bezeichnet. Am proximalen Ende des Steinfangkorbinstruments 1 ist eine Anschlusseinheit 4 vorgesehen, die ein erstes Anschlussstück 4a und ein gegenüber diesem axial relativbewegliches zweites Anschlussstück 4b umfasst. Das erste Anschlussstück 4a ist von hohlzylindrischer Form mit einem Kopfteil 5, der proximal mit einer Halbkugel 5a abschließt, an die sich distal ein Ringnutabschnitt 5b mit einer eingebrachten Ringnut 6 anschließt. Der Zugdraht 2 erstreckt sich mit seinem proximalen Endbereich durch die Hohlzylinderbohrung des ersten Anschlussstücks 4a hindurch bis zu dessen Kopfteil 5, mit dem er in einer geeigneten, herkömmlichen Weise z.B. durch Laserschweißen oder Kleben fest verbunden ist.

Am vorderen Ende ist das erste Anschlussstück 4a mit einem Kolben 7 versehen, mit dem es axial beweglich in einer Zylinderkammer 8 des zweiten Anschlussstücks 4b geführt ist. Mit anderen Worten sind die beiden Anschlussstücke 4a, 4b nach Art einer Kolben-Zylinder-Einheit gegeneinander axial beweglich. Eine in die Zylinderkammer 8 eingebrachte Schraubenfeder 9 spannt die beiden Anschlussstücke 4a, 4b in ihre in den Fig. 1A bis 1C gezeigte ausgefahrene Endstellung vor. Der Zugdraht 2 verläuft längsmittig durch das zweite Anschlussstück 4b hindurch, wobei letzteres über entsprechende stirnseitige Durchführungsbohrungen von der Zylinderkammer 8 nach außen verfügt. Der Schlauch 3 ist fest mit dem zweiten Anschlussstück 4b verbunden, speziell mit dessen vorderem Stirnendbereich, z.B. durch eine übliche Klebeverbindung oder ein anderes für diesen Zweck gebräuchliches Verbindungsmittel. In den zylindrischen Außenmantel des zweiten Anschlussstücks 4b ist eine Ringnut 10 eingebracht.

Die Fig. 2A bis 2C zeigen das Steinfangkorbinstrument in einem Zustand, in welchem ein am distalen Ende des Zugdrahts 2 vorgesehener Drahtkorb bzw. Steinfangkorb 11 vollständig vom Schlauch 3 freigegeben ist und sich daher in einem voll entfalteten Zustand befindet. In diesem Zustand befinden sich die beiden Anschlussstücke 4a, 4b in ihrer anderen, gegen die Kraft der Schraubenfeder 9 zusammengedrückten Endstellung, in welcher das erste Anschlussstück 4a maximal in das zweite Anschlussstück 4b eingeschoben ist.

Wenn ausgehend vom Zustand der Fig. 2A bis 2C die von einem Benutzer aufzubringende Bedienkraft, welche die beiden Anschlussstücke 4a, 4b gegen die Kraft der Schraubenfeder 9 zusammendrückt, wieder gelöst wird, drückt die Schraubenfeder 9 die beiden Anschlussstücke 4a, 4b wieder bis zur entgegengesetzten Endstellung gemäß den Fig. 1A bis 1C auseinander, in welcher der Drahtkorb 11 vollständig zusammengefaltet vom distalen Endabschnitt des Schlauchs 3 aufgenommen ist.

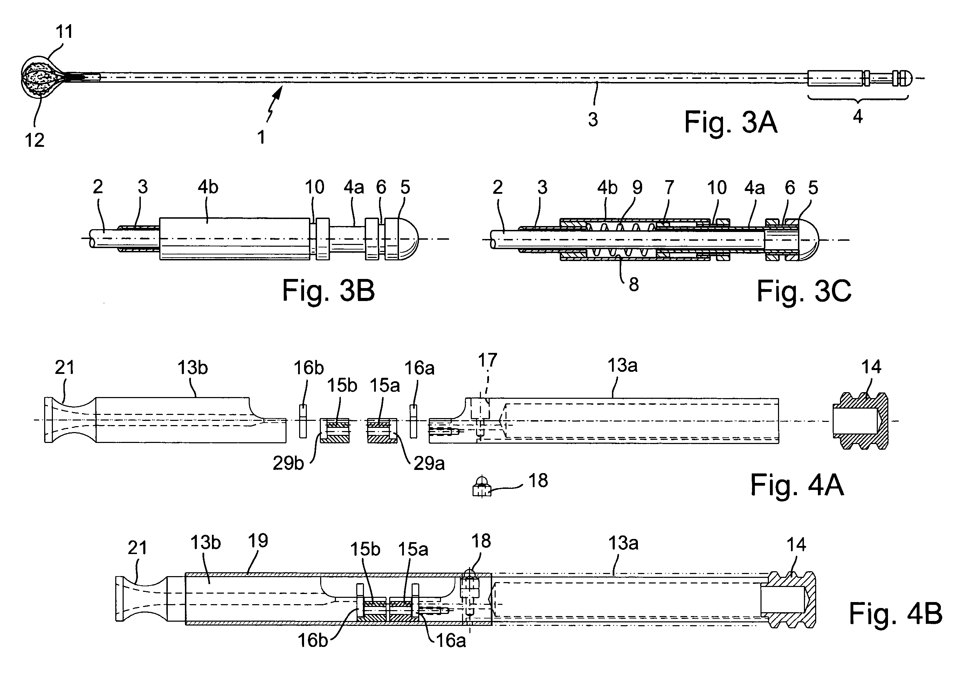

Wenn im Funktionsgebrauch des Steinfangkorbinstruments 1 ein Stein 12, wie in den Fig. 3A bis 3C veranschaulicht, durch den Drahtkorb 11 eingefangen wird und der Benutzer daraufhin das Einziehen des Drahtkorbs 11 freigibt, zieht sich der Drahtkorb 11 unter der Wirkung der Schraubenfeder 9 zusammen, bis er den Stein 12 klemmend festhält. Dies stellt den in den Fig. 3A bis 3C veranschaulichten Zustand des Steinfangkorbinstruments 1 dar.

Die Fig. 4A bis 4E zeigen ein Wechselgriffsystem mit einer Griffeinheit, mit der das Steinfangkorbinstrument 1 der Fig. 1 bis 3C mittels dessen Anschlusseinheit 4 lösbar gekoppelt werden kann, damit ein Benutzer durch Bedienen der Griffeinheit die oben erwähnten, üblichen Funktionsbewegungen des Steinfangkorbinstruments 1 ausführen kann, d.h. die Griffeinheit bildet im Zusammenwirken mit der Anschlusseinheit 4 ein Wechselgriffsystem für das Steinfangkorbinstrument 1.

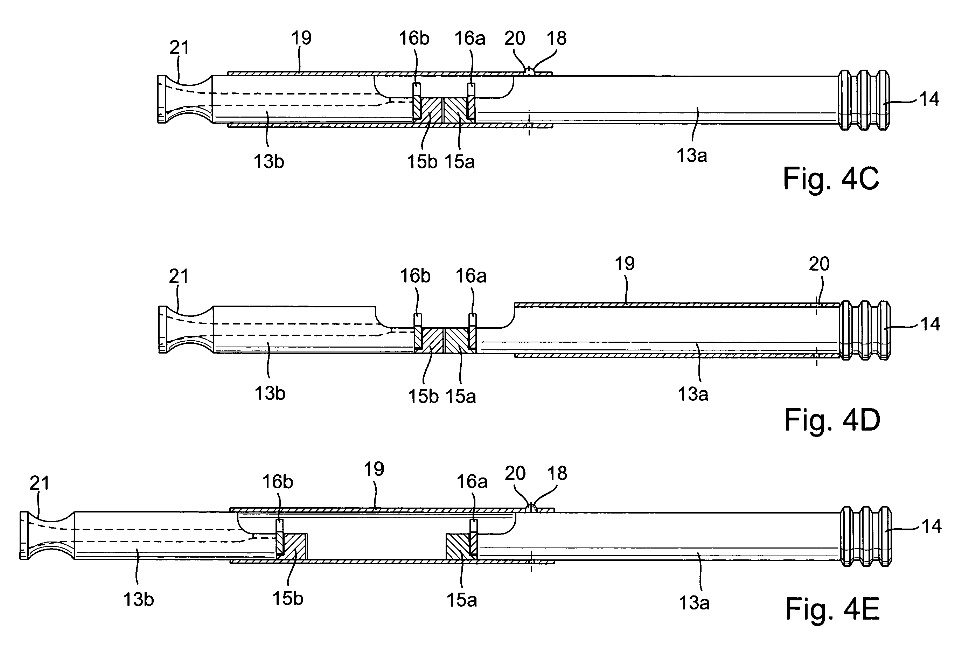

Wie aus der Explosionsdarstellung von Fig. 4A ersichtlich, umfasst die Griffeinheit einen ersten, hinteren Griffteil 13a, einen zweiten, vorderen Griffteil 13b, einen proximalen Abschlussstopfen 14, ein erstes Nutaufnahmestück 15a, ein zweites Nutaufnahmestück 15b, ein erstes Verriegelungselement 16a, ein zweites Verriegelungselement 16b und einen in eine Stiftaufnahme 17 im hinteren Griffteil 13a einzusetzenden Raststift 18 zum Verrasten eines in Fig. 4B in einer verrasteten vorderen Endstellung gezeigten Griffüberrohrs 19. Die Fig. 4B und 4C zeigen die Griffeinheit im montierten Zustand. Dazu wird das erste Nutaufnahmestück 15a am distalen Stirnende des hinteren Griffteils 13a befestigt, z.B. mittels einer Schraubverbindung, und das zweite Nutaufnahmestück 15b wird am proximalen Stirnende des vorderen Griffteils 13b fixiert, z.B. ebenfalls mittels einer Schraubverbindung. Das erste Sicherungs- bzw. Verriegelungselement 16a ist hierbei schwenkbeweglich in einer Aufnahme 29a des ersten Nutaufnahmestücks 15a zwischen letzterem und dem hinteren Griffteil 13a gehalten, und analog ist das zweite Sicherungs-/Verriegelungselement 16b in einer Aufnahme 29b des zweiten Nutaufnahmestücks 15b schwenkbeweglich zwischen letzterem und dem vorderen Griffteil 13b gehalten.

Die Fig. 4C und 4D zeigen die Griffeinheit in Seitenansicht, wobei lediglich das Griffüberrohr 19 zur besseren Erkennbarkeit der inneren Komponenten geschnitten dargestellt ist. Wie in den Fig. 4C und 4D veranschaulicht, ist das Griffüberrohr 19 am hinteren Griffteil 13a axial beweglich gehalten, wobei es in seiner vorderen Endstellung der Fig. 4C durch Eingriff des federelastisch nachgiebigen Raststifts bzw. Druckknopfs 18 in eine korrespondierende Rastöffnung 20 lösbar verrastet ist. In dieser Stellung überdeckt das Griffüberrohr 19 einen vorderen Endbereich des hinteren Griffteils 13a und einen hinteren Endbereich des vorderen Griffteils 13b einschließlich der Nutaufnahmestücke 15a, 15b und der Verriegelungselemente 16a, 16b. In einem vom Griffüberrohr 19 in seiner vorderen Endstellung nicht überdeckten, vorderen Abschnitt ist der vordere Griffteil 13b mit einer umfangsseitig umlaufenden Griffmulde 21 versehen. Durch radiales Eindrücken des Raststifts 18 kann die Verrastung des Griffüberrohrs 19 gelöst und letzteres anschließend zurückgeschoben werden, bis maximal in die hintere Endstellung von Fig. 4D. In dieser gibt es das vordere Griffteil 13b und einen vorderen Endbereich des hinteren Griffteils 13a einschließlich der Nutaufnahmestücke 15a, 15b und Verriegelungselemente 16a, 16b frei.

Wie in Fig. 4E veranschaulicht, sind die beiden Griffteile 13a, 13b axial relativbeweglich angeordnet, d.h. sie können ausgehend von der maximal zusammengeschobenen Stellung der Fig. 4B bis 4D axial auseinandergeschoben werden. Dabei sind sie mittels nicht näher gezeigter Führungsstäbe geführt, die sich längsverlaufend durch axiale Bohrungen hindurch erstrecken, die in den Nutaufnahmestücken 15a, 15b und/oder den Griffteilen 13a, 13b selbst vorgesehen sind. Zudem wird der vordere Griffteil 13b auch umfangsseitig durch das Griffüberrohr 19 geführt, wenn sich dieses in seiner verrasteten, vorderen Endstellung befindet, siehe die Fig. 4C und 4E.

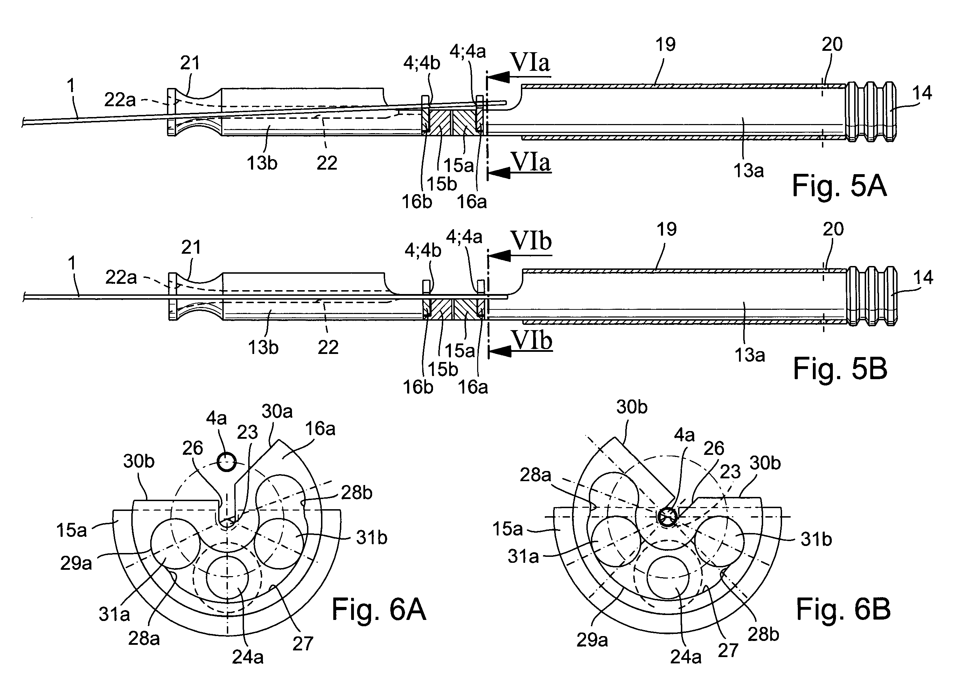

Die Fig. 5A und 5B veranschaulichen das Ankoppeln des Steinfangkorbinstruments der Fig. 1A bis 3C an die Griffeinheit der Fig. 4A bis 4E. Wie in Fig. 5A illustriert, wird dazu das Steinfangkorbinstrument 1 mit seiner proximalen Anschlusseinheit 4 voraus von vorn nach hinten durch eine Durchführungsöffnung 22 durchgeschoben, die als mittige Längsbohrung durch den vorderen Griffteil 13b hindurch eingebracht ist. Zum erleichterten Einführen des Steinfangkorbinstruments 1 weist die Durchführungsöffnung 22 eine sich nach vorn trichterförmig öffnende Aufweitung 22a auf. In einer beispielhaften Dimensionierung liegt der Außendurchmesser des Steinfangkorbinstruments 1 in der Größenordnung von ca. 1 mm, während die Durchführungsöffnung 22 einen geeignet größeren Durchmesser aufweist. Das Steinfangkorbinstrument 1 wird so weit in die Griffeinheit eingeschoben, bis sich die am ersten, proximalen Anschlussstück 4a vorgesehene Ringnut 6 auf Höhe des hinteren Verriegelungselements 16a befindet. Der Abstand der beiden Verriegelungselemente 16a, 16b in der Ankopplungsstellung von Fig. 5a, in welcher die beiden Griffteile 13a, 13b maximal zusammengeschoben sind, ist so gewählt, dass er gleich dem Abstand der beiden Ringnuten 6, 10 der Anschlusseinheit 4 in deren vorgespannter Endstellung gemäß den Fig. 1A bis 1C ist, die gleichzeitig die Ankopplungsmontagestellung des Steinfangkorbinstruments 1 ist. Dies hat zur Folge, dass sich die am vorderen Anschlussstück 4b vorgesehene Ringnut 10 auf Höhe des am vorderen Griffteil 13b angeordneten Verriegelungselements 16b befindet.

Nach Erreichen dieser Axialstellung des Steinfangkorbinstruments 1 gemäß Fig. 5A mit seiner Anschlusseinheit 4 auf Höhe des Bereichs der Nutaufnahmestücke 15a, 15b der Griffeinheit kann die Kopplung des Steinfangkorbinstruments 1 an die Griffeinheit erfolgen, indem mit leichtem Druck in Fig. 5A von oben die Anschlusseinheit 4 mit ihren Ringnuten 6, 10 in koppelnde, axial bewegungsstarre Wirkverbindung mit den Nutaufnahmestücken 15a, 15b und Sicherungselementen 16a, 16b und dadurch mit dem jeweiligen Griffteil 13a, 13b gebracht wird. Das Steinfangkorbinstrument 1 gelangt dadurch von einer leicht schrägen Einschublage gemäß Fig. 5A in seine exakt axiale Gebrauchslage gemäß Fig. 5B. Anschließend wird das so bewirkte Ankoppeln des hinteren Anschlussstücks 4a an das Nutaufnahmestück 15a des hinteren Griffteils 13a durch das Verriegelungselement 16a gegen unbeabsichtigtes Lösen verriegelt, und ebenso wird das Ankoppeln des vorderen Anschlussstücks 4b an das Nutaufnahmestück 15b des vorderen Griffteils 13b durch das Verriegelungselement 16b gegen unbeabsichtigtes Lösen gesichert. Auf diese Weise ist der Zugdraht 2 des Steinfangkorbinstruments 1 über das hintere Anschlussstück 4a axial bewegungsstarr und lösbar mit dem hinteren Griffteil 13a gekoppelt, und der Schlauch 3 des Steinfangkorbinstruments 1 ist über das vordere Anschlussstück 4b der Anschlusseinheit 4 axial bewegungsstarr mit dem vorderen Griffteil 13b gekoppelt.

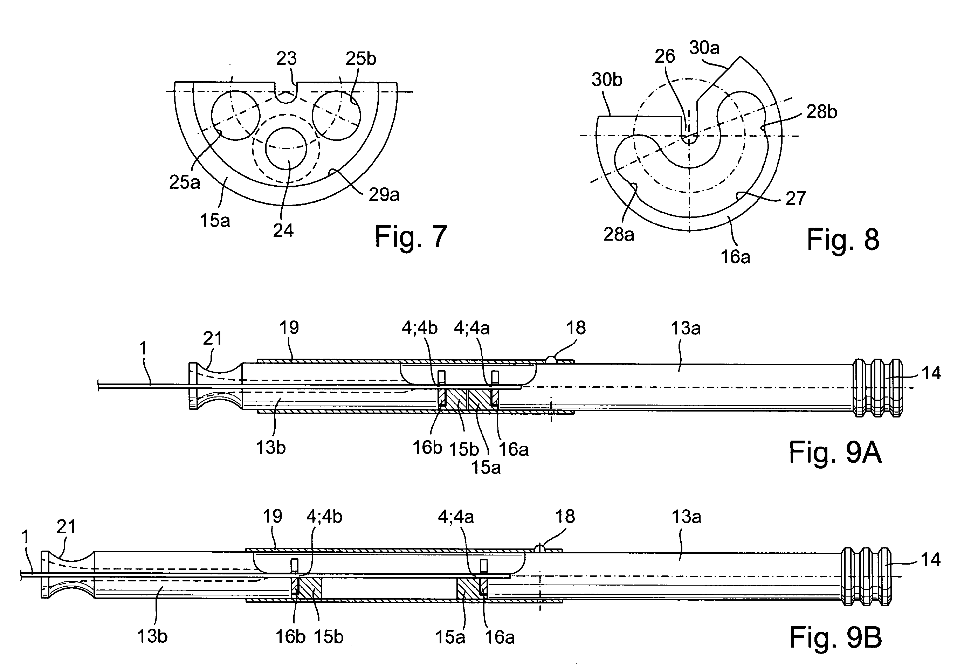

Die Fig. 6A und 6B veranschaulichen in Verbindung mit den Einzeldarstellungen von Fig. 7 und Fig. 8 genauer das verriegelbare Ankoppeln am Beispiel der Koppelverbindung des hinteren Anschlussstück 4a am hinteren Griffteil 13a des Nutaufnahmestücks 15a und des Verriegelungselements 16a. Wie aus Fig. 7 ersichtlich, weist das Nutaufnahmestück 15a (und in gleicher Weise das andere Nutaufnahmestück 15b) eine etwa halbzylindrische Gestalt mit einem mittigen, axialen Aufnahmeschlitz 23 auf, in welchen das Steinfangkorbinstrument 1 mit seinem entsprechenden Abschnitt der Anschlusseinheit 4 aufnehmbar ist. Eine axiale Schrauböffnung 24 dient zum Durchführen eines Schraubbolzens 24a, mit dem das Nutaufnahmestück 15a am betreffenden Stirnende des zugehörigen Griffteils 13a befestigt wird. Des Weiteren sind in das Nutaufnahmestück 15a zwei axiale Durchführungsöffnungen 25a, 25b eingebracht, durch die jeweils ein Führungsstab 31 a, 31 b hindurchgeführt ist, um die beiden Griffteile 13a, 13b axial beweglich aneinander zu führen, wie oben erwähnt.

Wie aus Fig. 8 ersichtlich, ist das Verriegelungselement 16a (und in gleicher Weise das andere Verriegelungselement 16b) als scheibenförmiges Element mit einer offenen, sich über etwas weniger als 270° erstreckendes Scheibenelement mit einem mittigen, axialen Aufnahmeschlitz 26 gebildet, der als eine Nutaufnahme für die korrespondierende, aufzunehmende Ringnut 6 des anzukoppelnden Anschlussstücks 4a des Steinfangkorbinstruments 1 fungiert. Dies bedeutet, dass die Weite des Aufnahmeschlitzes 26 so gewählt ist, dass sie in etwa dem Außendurchmesser des Anschlussstücks 4a im Bereich der Ringnut 6 entspricht oder nur geringfügig größer ist, jedoch kleiner als der Außendurchmesser des Anschlussstücks 4a im Bereich des Kopfteilabschnitts 5b außerhalb der Ringnut 6. Zudem entspricht die axiale Länge des Verriegelungselements 16a derjenigen der Ringnut 6 oder ist allenfalls geringfügig kleiner. Des Weiteren ist das Verriegelungselement 16a mit einem halbringförmigen Führungsschlitz 27 versehen, dessen Weite in etwa dem Durchmesser der Führungsstab-Durchführungsöffnungen 25a, 25b des Nutaufnahmestücks 15a und dem entsprechenden Durchmesser der durchgeführten Führungsstäbe 31 a, 31 b entspricht, wodurch das Verriegelungselement 16a in einem durch die Winkelausdehnung des Führungsschlitzes 27 bestimmten Maß schwenkbeweglich in der korrespondierenden Aufnahme 19a mittels der Führungsstäbe 31 a, 31 b geführt ist. An beiden Endbereichen ist der Führungsschlitz 27 mit je einer Engstelle 28a, 28b versehen, wodurch eine Verrastung des Verriegelungselements 16a in jeder seiner beiden Endstellungen, wie sie in den Fig. 6A und 6B gezeigt sind, an je einem der beiden Führungsstäbe 31 a, 31 b bewirkt wird.

In der Montageposition von Fig. 6A sind der Anschlussstück-Aufnahmeschlitz 23 des Nutaufnahmestücks 15a und der Nutaufnahmeschlitz 26 des Verriegelungselements 16a beide nach oben geöffnet ausgerichtet. Durch die oben geschilderte Eindrückbewegung gelangt das zuvor gemäß den Fig. 5A und 6A über den beiden Schlitzen 23, 26 liegende Anschlussstück 4a in seine zentrische Position in den Schlitzen 23, 26, wobei die Ringnut 6 in den Nutaufnahmeschlitz 26 gelangt und dadurch das Anschlussstück 4a und folglich der an ihm fixierte Zugdraht 2 axial bewegungsstarr mit dem zugeordneten Griffteil 13a gekoppelt ist. Durch Verschwenken des Verriegelungselements 16a von seiner Montage-/Entriegelungsposition gemäß Fig. 6A ist die geschilderte Koppelverbindung dann gegen Lösen gesichert, indem sich der Nutaufnahmeschlitz 26 gegenüber dem stationär bleibenden Anschlussstück-Aufnahmeschlitz 23 verdreht und die gemeinsame, überlappende Öffnungsweite beider Schlitze 23, 26 dadurch auf einen Wert kleiner als der Durchmesser der Ringnut 6 verringert wird. Damit ist der Zugdraht 2 des Steinfangkorbinstruments 1 über sein zugehöriges Anschlussstück 4a verriegelt am hinteren Griffteil 13a gehalten. Zum Lösen der Koppelverbindung wird das Verriegelungselement 16a von seiner Verriegelungsposition gemäß Fig. 6B in seine entriegelnde Position gemäß Fig. 6A zurückgeschwenkt. Das verriegelnde und entriegelnde Bedienen das Verriegelungselements 16a wird durch die offene Scheibenform erleichtert, deren beide Endflächen 30a, 30b jeweilige Bedienflächen bilden, auf die vom Benutzer zum Bewirken der verriegelnden bzw. entriegelnden Schwenkbewegung des Verriegelungselements 16a gedrückt wird.

Es versteht sich, dass in gleicher Weise das lösbare Ankoppeln des Schlauchs 3 des Steinfangkorbinstruments 1 an den vorderen Griffteil 13b über das vordere Anschlussstück 4b und die zugehörigen Kopplungsmittel erfolgt, d.h. über das Nutaufnahmestück 15b und das in dessen Aufnahme 19b schwenkbeweglich angeordnete Verriegelungselement 16b. Dies beinhaltet insbesondere das Einlegen des vorderen Anschlussstücks 4b in den zugehörigen Aufnahmeschlitz des Nutaufnahmestücks 15b und dabei von dessen Ringnut 10 in den Nutaufnahmeschlitz des Verriegelungselements 16b gleichzeitig mit dem oben beschriebenen Einlegen des hinteren Anschlussstücks 4a in die korrespondierenden Schlitze 23, 26 des Nutaufnahmestücks 15a bzw. des Verriegelungselements 16a und das anschließende Verriegeln dieser Koppelverbindung durch entsprechendes Verdrehen des Verriegelungselements 16b. So nimmt das Steinfangkorbinstrument 1 mit dem Zugdraht 2 und dem Schlauch 3 seine in Fig. 5B gezeigte Gebrauchslage ein, in welcher der Zugdraht 2 lösbar verriegelt axial bewegungsstarr mit dem hinteren Griffteil 13a und der Schlauch 3 in gleicher Weise lösbar verriegelt axial bewegungsstarr mit dem vorderen Griffteil 13b gekoppelt ist.

Es versteht sich, dass alternativ zu den Kopplungs-/Sicherungsmitteln gemäß den Figuren 6A bis 8 beliebige andere herkömmliche Kopplungs/Sicherungsmittel verwendbar sind, wie sie der Fachmann zur Erfüllung der geschilderten Kopplungs- und Sicherungsfunktionalitäten in einer herkömmlichen Weise unter Verwendung von kraftschlüssigen und/oder formschlüssigen Verbindungen realisieren kann. So kann die Anordnung von Nut einerseits und Nutaufnahme bzw. Nuteingriffselement andererseits am betreffenden Anschlussstück bzw. am betreffenden Griffteil gegenüber den oben beschriebenen Varianten vertauscht sein. Zur Sicherung bzw. Verriegelung der lösbaren Nut-Nuteingriffselement-Verbindung sind statt der oben erläuterten Drehbewegung des jeweiligen Verriegelungselements 16a, 16b andere Sicherungsbewegungen mit entsprechend modifizierten Verriegelungselementen einsetzbar, z.B. das Anordnen eines schlitzabdeckenden Verriegelungselementes, das um eine zur Griffteillängsachse senkrechte Querachse drehbeweglich ist, um wahlweise die Aufnahmenut mit aufgenommenem Nuteingriffselement abzudecken oder freizugeben. Weitere Lösungsvarianten sind ein Blockierelement, das um eine zur Griffteillängsachse parallele, aber versetzte Kippachse zwischen einer Nutabdeckposition und einer Freigabeposition verschwenkbar ist, ein Blockierelement, das in ähnlicher Weise um eine zur Griffteillängsachse senkrechte Querachse kippbeweglich ist, oder ein translatorisch zwischen Blockierposition und Freigabeposition bewegliches Blockierschieberelement.

Für die drehbewegliche Fixierung sind beliebige herkömmliche Mittel verwendbar, wie Kugelscharniere, Filmscharniere oder Steckachsen. Die Fixierung des jeweiligen Verriegelungselements in seiner Verriegelungsstellung kann z.B. durch das Griffüberrohr 19 realisiert sein. Dies verhindert ein unabsichtliches Entriegeln ohne weitere Hilfsmittel. Beispielsweise kann diese Fixierung der Verriegelung so gestaltet sein, dass das Verriegelungselement in der hinteren Stellung des Griffüberrohrs 19 zwischen seiner Blockierstellung und seiner Freigabestellung frei beweglich ist, während es bei vorgeschobenem Griffüberrohr 19 nicht mehr aus seiner Blockierstellung in die Freigabestellung bewegbar ist.

Die Fig. 9A und 9B veranschaulichen die Bedienung und Funktionsweise des solchermaßen gebrauchsfertig mit der Griffeinheit gekoppelten Steinfangkorbinstruments 1. In der Griffposition von Fig. 9A mit den beiden maximal zusammengeschobenen Griffteilen 13a, 13b befinden sich entsprechend die beiden Anschlussstücke 4a, 4b der Anschlusseinheit 4 in ihrer maximal zusammengeschobenen bzw. zusammengedrückten Stellung gemäß den Fig. 2B und 2C, d.h. das Steinfangkorbinstrument 1 befindet sich in seinem Zustand mit vollständig ausgefahrenem Steinfangkorb 11 gemäß Fig. 2A. Im Funktionszustand von Fig. 9B mit maximal auseinandergeschobenen Griffteilen 13a, 13b befinden sich entsprechend die beiden Anschlussstücke 4a, 4b der Anschlusseinheit 4 in ihrer maximal auseinandergezogenen Stellung gemäß den Fig. 1B und 1C, d.h. das Steinfangkorbinstrument 1 befindet sich in seinem Zustand mit vollständig in den Schlauch 3 eingefahrenem Steinfangkorb 11 gemäß Fig. 1A. Vorteilhafterweise ist dies der Funktionszustand, den das Steinfangkorbinstrument 1 von selbst einnimmt, d.h. ohne aktive Benutzerbetätigung.

Das Steinfangkorbinstrument 1 kann folglich ohne aktive, die beiden Griffteile 13a, 13b relativ zueinander bewegende Benutzerbetätigung im Zustand von Fig. 1A mit vollständig im Schlauch 3 aufgenommenem Steinfangkorb 11 in einen Körpergewebekanal eingeführt werden. Wenn sich das distale Ende des Steinfangkorbinstruments 1 am beabsichtigten Gebrauchsort befindet, d.h. in der Nähe eines einzufangenden Steins oder dergleichen, betätigt der Benutzer die Griffeinheit, indem er die beiden Griffteile 13a, 13b von ihrer ausgezogenen Stellung gemäß Fig. 9B in ihre zusammengeschobene Stellung gemäß Fig. 9A verbringt. Dadurch gelangt der Steinfangkorb 11 aus dem Schlauch 3 heraus und nimmt seine aufgefaltete Position gemäß Fig. 2A ein.

Sobald ein Stein, Partikel oder dergleichen im Drahtkorb 11 eingefangen wurde, kann er darin festgeklemmt werden, indem der Benutzer den vorderen Griffteil 13b axial vorbewegt, bis der Stein 12 durch die dadurch verursachte Drahtkorbeinzugsbewegung vom Drahtkorb 11 klemmend festgehalten wird, d.h. das Steinfangkorbinstrument 1 befindet sich dann im Zustand der Fig. 3A bis 3C. Die Vorspannungswirkung der Schraubenfeder 9 in der Anschlusseinheit 4 hält die beiden Anschlussstücke 4a, 4b und folglich die damit jeweils axial bewegungsstarr gekoppelten Griffteile 13a, 13b axial auseinandergedrückt und sorgt so dafür, dass der Stein 12 selbsttätig vom Drahtkorb 11 festgeklemmt bleibt. Das Steinfangkorbinstrument 1 kann daraufhin mit dem distal im Drahtkorb 11 eingefangenen und von diesem klemmend festgehaltenen Stein 12 aus dem Körpergewebekanal herausbewegt werden. Wiederum ist dazu wie beim Einführen des Steinfangkorbinstruments 1 in den Körpergewebekanal keine aktive Bedienbetätigung der beiden Griffteile 13a, 13b relativ zueinander durch den Benutzer erforderlich. Denn wie bei der Einführungsbewegung hält die Schraubenfeder 9 der Anschlusseinheit 4 das Steinfangkorbinstrument 1 selbsttätig in der gewünschten Relativposition von Zugdraht 2 und Schlauch 3, beim Einführen (und natürlich auch beim etwaigen Herausbewegen ohne eingefangenen Gegenstand) in der Position von Fig. 1A mit vollständig in den Schlauch 3 eingezogenem Drahtkorb 11 und beim Herausbewegen mit dem Stein 12 in der Position gemäß Fig. 3A.

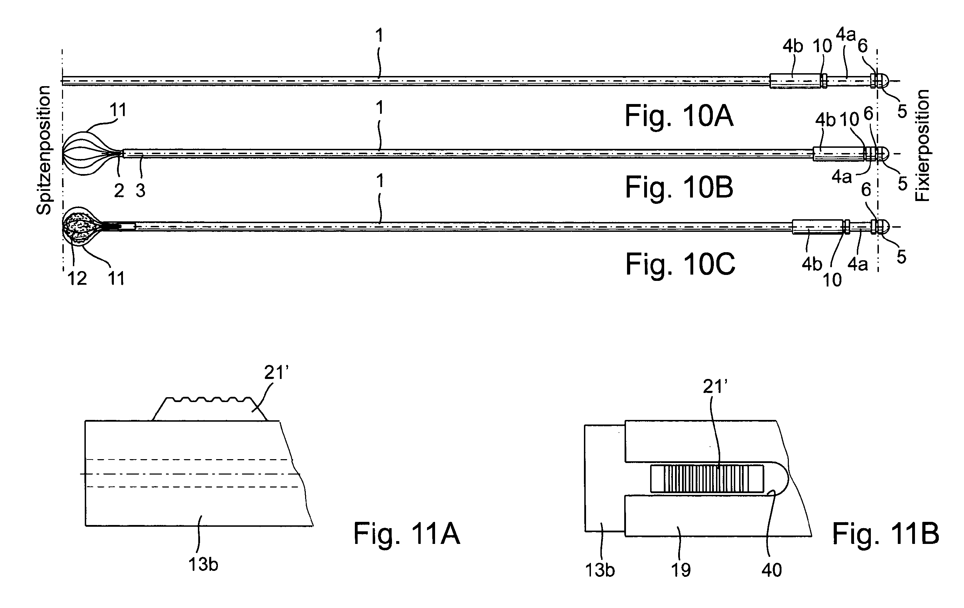

In den Fig. 10A bis 10C ist ein weiterer Vorteil der Verwendung des beschriebenen Wechselgriffsystems in Anwendung auf das Steinfangkorbinstrument 1 veranschaulicht. Die Griffeinheit mit den beiden Griffteilen 13a, 13b ist dazu so ausgelegt, dass die oben zu den Fig. 9A und 9B geschilderte Funktionsbewegung der beiden Griffteile 13a, 13b relativ zueinander vorzugsweise dadurch bewirkt wird, dass der Benutzer das vordere Griffteil 13b aktiv betätigt, d.h. axial bewegt, während das hintere Griffteil 13a im Wesentlichen stationär gehalten werden kann. Das aktive Betätigen des vorderen Griffteils 13b wird durch die Griffmulde 21 erleichtert, an welcher der Benutzer das Griffteil 13b zur Betätigung greifen kann. Dies hat zur Folge, dass nach dem Einführen des Steinfangkorbinstruments 1 in einen Körpergewebekanal der mit dem hinteren Griffteil 13a axial bewegungsstarr verbundene Zugdraht seine Position im Körpergewebekanal beibehält, während sich der Steinfangkorb 11 aus dem Schlauch 3 herausbewegt und sich auffaltet, indem der axial bewegungsstarr mit dem vorderen Griffteil 13b gekoppelte Schlauch 3 durch das Zurückbewegen des vorderen Griffteils 13b axial relativ zum Körpergewebekanal und zum Zugdraht 2 und dem an dessen distalem Ende vorgesehenen Drahtkorb 11 zurückgezogen wird. Dies bedeutet, dass der Steinfangkorb 11 während seines Entfaltens im Körpergewebekanal an Ort und Stelle verbleibt und sich sein distales Ende und damit dasjenige des Steinfangkorbinstruments 1 nicht merklich im Körpergewebekanal axial verschiebt (bis auf eine etwaige geringfügige axiale Verschiebung des distalen Endes des Drahtkorbs 11, die durch die Korbauffaltbewegung bedingt sein kann). Dies ist in den Fig. 10A und 10B durch die im Wesentlichen gleichbleibende distale Endposition bzw. Spitzenposition und die im Wesentlichen gleichbleibende Position des mit dem hinteren Griffteil 13a gekoppelten hinteren Anschlussstücks 4a (Fixierposition) illustriert. Lediglich das vordere Anschlussstück 4b und der mit ihm gekoppelte Schlauch 3 führen eine aktive axiale Bewegung aus.

Wie in Fig. 10C veranschaulicht, bleibt diese konstante distale Endposition (Spitzenposition) und proximale Endposition (Fixierposition) des mit dem erfindungsgemäßen Wechselgriffsystem gekoppelten Steinfangkorbinstruments 1 auch beim Einfangen und Festklemmen eines Gegenstands, wie des Steins 12, im Wesentlichen erhalten. Dazu wird das hintere Griffteil 13a in der Fixierposition gehalten, z.B. durch Ankoppeln an eine externe stationäre Einheit oder durch außenseitiges Fixieren am Körper des Patienten beispielsweise mittels Pflaster oder durch einfaches benutzerseitiges Festhalten. Durch axiales Vorwärtsbewegen des vorderen Griffteils 13b und damit des vorderen Anschlussstücks 4b mit dem Schlauch 3 relativ zum hinteren Anschlussstück 4a und dem hinteren Griffteil 13a mit dem Zugdraht 2 wird der Drahtkorb 12 wieder in den Schlauch 3 eingezogen, bis er den eingefangenen Gegenstand klemmend festhält. Die Vorspannung der Schraubenfeder 9 sorgt selbsttätig dafür, dass der eingefangene Gegenstand zuverlässig festgeklemmt bleibt, auch wenn der Benutzer den vorderen Griffteil 13b bzw. die Griffeinheit anschließend loslässt. Dabei ändert der Drahtkorb, wie gesagt, seine axiale Lage nicht oder jedenfalls nicht wesentlich.

Diese in den Fig. 10A bis 10C veranschaulichte Funktionalität ist für ein einfaches und zuverlässiges Einfangen, Festhalten und Entfernen von Steinen oder dergleichen durch das Steinfangkorbinstrument 1 von großem Vorteil. Denn der Drahtkorb 12 kann vergleichsweise genau in axiale Höhe des einzufangenden Gegenstands gebracht und auf dieser axialen Höhe während der Steineinfangbewegung gehalten werden, im Unterschied zu solchen herkömmlichen Steinfangkorbinstrumenten, bei denen das Auffalten und Zusammenziehen des Steinfangkorbs im Körpergewebekanal gleichzeitig von einer axialen Bewegung des Zugdrahts und damit auch des Steinfangkorbs begleitet ist.

Die obige Beschreibung macht deutlich, dass und wie das Steinfangkorbinstrument 1 sehr leicht und zuverlässig von der Griffeinheit mit den beiden Griffteilen 13a, 13b abgenommen werden kann, wenn dies erforderlich ist, z.B. während das Steinfangkorbinstrument 1 in einen Körpergewebekanal eingeführt ist. Dazu wird ausgehend von der Funktionsposition gemäß Fig. 10A oder 10C und den zugehörigen Griffpositionen, wie oben zu den Fig. 9A und 9B erläutert, das Griffüberrohr 19 nach Lösen der Verrastung 18 in seine axial hintere Endposition verbracht, wonach die beiden Verriegelungselemente 16a, 16b von ihrer Verriegelungsposition in ihre Entriegelungsposition verschwenkt werden. Daraufhin können die beiden Anschlussstücke 4a, 4b aus ihren Aufnahmeschlitzen in den Nutaufnahmestücken 15a, 15b und Verriegelungselementen 16a, 16b radial herausbewegt werden, bis ihre jeweilige Ringnutverrastung gelöst ist. Dann lassen sich die beiden Griffteile 13a, 13b in proximaler Richtung vom Steinfangkorbinstrument 1 abziehen.

Je nach Bedarf kann dann eine andere Bedien-/Betätigungseinheit an das Steinfangkorbinstrument 1 angekoppelt werden, oder das Steinfangkorbinstrument 1 kann zu einem späteren Zeitpunkt ohne Zuhilfenahme der Griffeinheit aus dem Körpergewebekanal herausgezogen werden. Wenn sich das Steinfangkorbinstrument 1 innerhalb eines Katheterschlauchs oder Endoskops befindet, kann nach Abnehmen der Griffeinheit z.B. auch nur der Katheterschlauch aus dem Körpergewebe herausgezogen werden, während das Steinfangkorbinstrument 1 darin verbleibt. Die Anschlusseinheit 4a, 4b des Steinfangkorbinstruments 1 ist mit so geringem Außendurchmesser ausgelegt, dass der Katheterschlauch oder das Endoskop über sie hinweg herausgezogen werden kann, ohne dass die Anschlusseinheit 4a, 4b dazu vom Zugdraht 2 und vom Schlauch 3 abgetrennt werden muss. Bei Bedarf kann die Griffeinheit mit den beiden Griffteilen 13a, 13b zu einem späteren Zeitpunkt auch wieder an das Steinfangkorbinstrument 1 angekoppelt werden, wie oben erläutert. Das gleiche Lösen der Koppelverbindung des Steinfangkorbinstruments 1 mit der Griffeinheit ist selbstverständlich möglich, wenn sich das Steinfangkorbinstrument 1 außerhalb eines Körpergewebekanals befindet, beispielsweise nachdem es aus dem Körpergewebekanal herausgezogen wurde und dabei mit der Griffeinheit gekoppelt blieb. Die Griffeinheit mit den beiden Griffteilen 13a, 13b kann dann beispielsweise zum Ankoppeln eines nächsten Steinfangkorbinstruments verwendet werden.

Die Figuren 11A und 11B veranschaulichen eine Variante des Instruments gemäß den Fig. 1A bis 10C, bei der als einzigem Unterschied anstelle der Griffmulde 21 als Bedienbetätigungsmittel ein Schieber 21' am entsprechenden vorderen Endbereich des vorderen Griffteils 13b vorgesehen ist. Das Griffüberrohr 19 ist mit einer korrespondierenden frontseitigen Schlitzausnehmung 40 versehen, in welcher der Schieber 21' in der gezeigten Stellung des vorderen Griffteils 13b aufgenommen ist, in der sich dieses weitestgehend im Griffüberrohr 19 befindet. Dies ermöglicht eine gegenüber der Griffmuldenvariante verkürzte Ausführung des vorderen Griffteils 13b und damit der gesamten Griffeinheit. Zudem ist der Schieber 21' in seiner Form frei z.B. nach optimalen ergonomischen Gesichtspunkten gestaltbar.

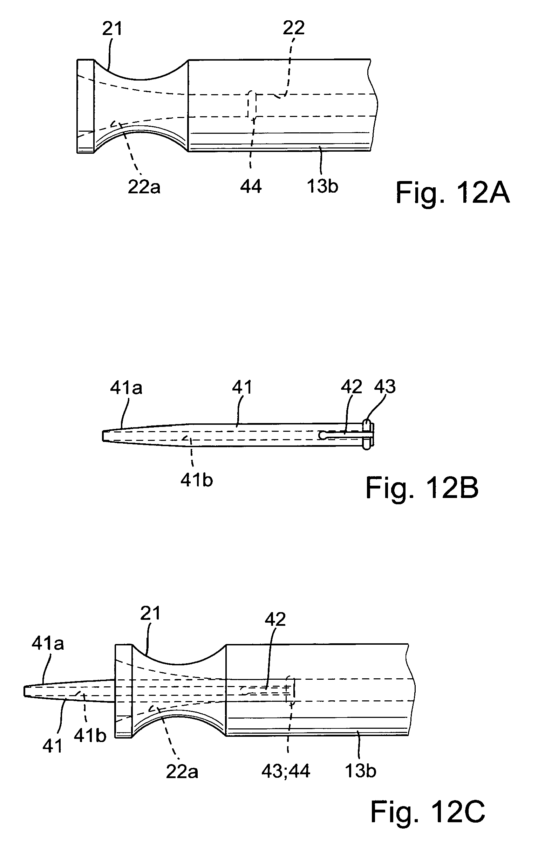

Die Fig. 12A bis 12C veranschaulichen eine Variante, bei der das Steinfangkorbinstrument zusätzlich über eine Einführhilfshülse 41 verfügt, die lösbar am vorderen Endbereich des hierfür entsprechend modifizierten vorderen Griffteils 13b angebracht werden kann. Zu diesem Zweck weist die Einführhilfshülse 41 an ihrem hinteren Endbereich eine Schlitzung 42 und eine radiale Wulst 43 auf. Korrespondierend dazu ist die Durchführungsöffnung 22 am vorderen Griffteil 13b hinter ihrer trichterförmigen Aufweitung 22a mit einer ringförmigen Rastnut 44 versehen. Die Einführhilfshülse 43 kann auf diese Weise mit ihrem hinteren Ende in die Trichteröffnung 22a des vorderen Griffteils 13b eingeschoben werden. Ihr hinterer Endbereich drückt sich nach Passieren der trichterförmigen Aufweitung 22a leicht zusammen, was durch die Schlitzung 42 ermöglicht wird, und federt dann rastend wieder radial nach außen zurück, sobald die Ringwulst 43 in den Bereich der Rastnut 44 gelangt, um dann die in Fig. 12C gezeigte, lösbar eingerastete Position einzunehmen.

Die Einführhilfshülse 41 dient dazu, das Einführen des Zugdrahts 2 mit dem daran distal angeordneten Drahtkörbchen 11 und des das Drahtkörbchen 11 umschließenden Schlauchs 3 in den Arbeitskanal eines Endoskops zu erleichtern und so auch das empfindliche Drahtkörbchen 11 zusätzlich zu schützen. Dazu wird die Einführhilfshülse 41, nachdem das Steinfangkorbinstrument 1 in der oben beschriebenen Weise mit der Griffeinheit 13a, 13b gekoppelt worden ist, über den Schlauch 3 bis zum distalen Ende von Schlauch 3 und Zugdraht 2 nach vorn geschoben. Dazu wird sie unter leichtem Zug aus ihrer Verrastung gemäß Fig. 12C gelöst und dann aus dem vorderen Griffteil 13b heraus nach vorn bewegt. Entsprechend ihrer Funktion als Hilfshülse beim Einführen in den Arbeitskanal eines Endoskops weist die Einführhilfshülse 41 einen stumpfen konischen vorderen Endbereich 41 a auf. Ein durchgehender Längskanal 41 b der Einführhilfshülse 41 ist in seinem Durchmesser angepasst an den Außendurchmesser des Schlauchs 3 geeignet gewählt, um das besagte Verschieben der Einführhilfshülse 41 über den Schlauch 3 hinweg zu erlauben. Beim Ankoppeln des Steinfanginstruments 1 an die Griffeinheit 13a, 13b wird selbiges durch den Durchführungskanal 41 b der Einführhilfshülse 41 durchgeführt, entweder bevor die Einführhilfshülse 41 mit dem vorderen Griffteil 13b verrastet wird oder alternativ danach.

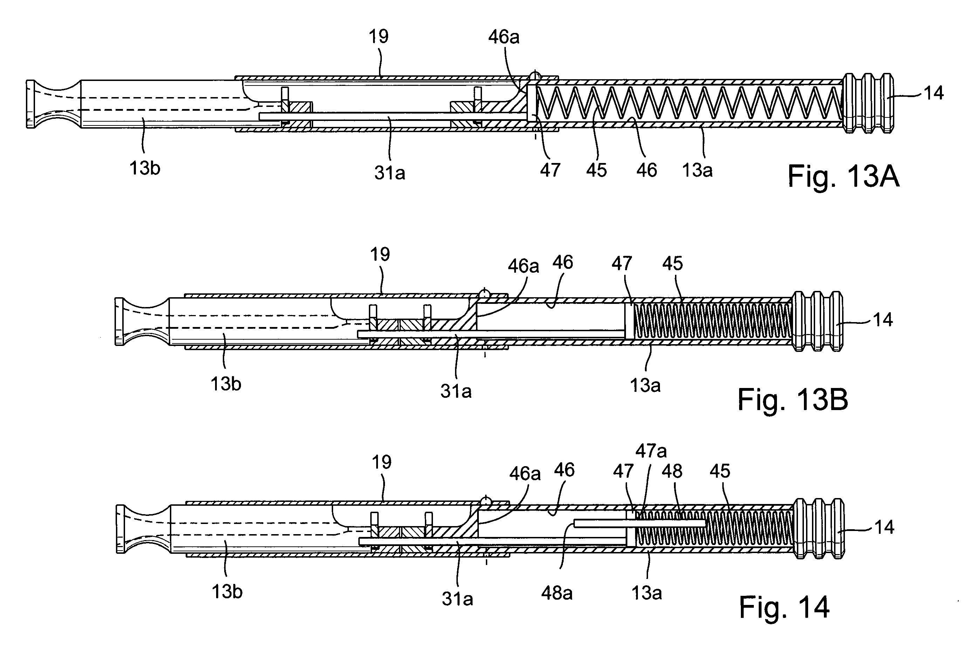

Die Figuren 13A und 13B veranschaulichen eine Variante der Griffeinheit, die sich von der zuvor beschriebenen durch das zusätzliche Vorhandensein eines elastischen Vorspannelements unterscheidet, hier beispielhaft in Form einer Schraubenfeder 45, die in einer korrespondierenden axialen Aufnahmebohrung 46 des hinteren Griffteils 13a aufgenommen ist. Am hinteren Ende stützt sich die Schraubenfeder 45 gegen den Abschlussstopfen 14 ab, an ihrem vorderen Ende gegen eine Anschlagscheibe 47, die am hinteren Ende wenigstens eines der Führungsstäbe 31 a, 31 b fixiert ist.

Die Schraubenfeder 45 spannt die beiden Griffteile 13a, 13b in ihre ausgezogene Endstellung gemäß Fig. 13a vor. Dabei kommt die Anschlagscheibe 47 gegen eine Bodenfläche 46a der Aufnahmebohrung 46 des hinteren Griffteils 13a zur Anlage, was diese Endstellung definiert. Aus dieser ausgezogenen Endstellung können die beiden Griffteile 13a, 13b gegen die Kraft der Schraubenfeder 45 in ihre andere Endstellung gemäß Fig. 13B zusammengedrückt werden, indem das vordere Griffteil 13b in das Griffüberrohr 19 eingeschoben wird. Die Schraubenfeder 45 besitzt folglich die gleiche Wirkrichtung wie die Schraubenfeder 9 in der Anschlusseinheit 4 des Steinfangkorbinstruments 1 und unterstützt diese somit in deren Funktion, wie sie oben erläutert wurde, worauf verwiesen werden kann. Es versteht sich, dass je nach Bedarf und Anwendungsfall anstelle der Schraubenfeder 9 der Anschlusseinheit 4 und/oder der Schraubenfeder 45 der Griffeinheit 13a, 13b andere elastische Elemente einsetzbar sind, und zwar statt der gezeigten druckelastischen Elemente alternativ auch zugelastische Elemente, wobei ggf. auch unterschiedliche Wirkrichtungen für die beiden elastischen Elemente vorgesehen sein können und/oder eines der beiden elastischen Elemente auf Druck und das andere auf Zug beansprucht werden kann.

Fig. 14 zeigt eine weitere Variante ausgehend vom Ausführungsbeispiel der Fig. 13A und 13B, wobei das Ausführungsbeispiel von Fig. 14 als einzigen Unterschied zusätzlich ein variables Hubbegrenzungselement in Form eines hubbegrenzenden Längsstabs 48 umfasst, der wie gezeigt in einer dazu in der Anschlagscheibe 47 eingebrachten Durchführungsbohrung 47a längsverstellbar fixiert ist. Die auseinandergezogene Endstellung der beiden Griffteile 13a, 13b wird in diesem Fall dadurch definiert, dass der Hubbegrenzungsstab 48 mit seinem Vorderende 48a gegen den Boden 46a des Federaufnahmeraums 46 des hinteren Griffteils 13a zur Anlage kommt. Diese Endstellung kann durch Längsverstellung des Hubbegrenzungsstabs 48 an der Anschlagscheibe 47 variabel eingestellt werden. Damit kann die Griffeinheit 13a, 13b für Steinfangkorbinstrumente 1 mit Drahtkörben 11 unterschiedlicher Länge verwendet und an die jeweils vorliegende Drahtkorblänge optimal angepasst werden. Es versteht sich, dass eine solche variable Hubbegrenzung der axialen Relativbeweglichkeit der beiden Griffteile 13a, 13b alternativ zum gezeigten Hubbegrenzungsstab 48 auch auf eine beliebige andere herkömmliche Weise realisierbar ist. Des Weiteren kann bei Bedarf auch die andere, zusammengeschobene Endstellung der beiden Griffteile 13a, 13b derart definiert werden, indem beispielsweise ein zugehöriger Hubbegrenzungsstab geeigneter Länge am Abschlussstopfen 14 fixiert wird, gegen den dann die Anschlagscheibe 47 zur Anlage kommt. Alternativ kann der Hubbegrenzungsstab 48 in seiner Position und Länge so gewählt sein, dass er zur Definition beider Endstellungen der Griffteile 13a, 13b dient.

Wie die obige Beschreibung deutlich macht, ermöglicht das dort erläuterte Wechselgriffsystem ein vergleichsweise einfaches und komfortables, lösbares Ankoppeln eines Steinfangkorbinstruments, das hierfür mit einer geeigneten Anschlusseinheit ausgerüstet ist. Montage und Demontage des Steinfangkorbinstruments an bzw. von der Griffeinheit mit den beiden Griffteilen erfolgt über die Anschlusseinheit mit den beiden Anschlussstücken ohne lose Teile und ohne zusätzliche Hilfsmittel, wie Werkzeug. Die geschilderte Koppelverbindung sorgt für eine axial bewegungsstarre Verbindung zwischen Steinfangkorbinstrument und Wechselgriffeinheit, genauer zwischen dem Zugdraht mit dem hinteren Griffteil über das hintere Anschlussstück sowie des Schlauchs mit dem vorderen Griffteil über das vordere Anschlussstück, allein durch jeweiligen Formschluss der Nuten an den Anschlussstücken mit den Aufnahmeschlitzen der Nutaufnahmestücke und Verriegelungselemente. Dabei ermöglichen die Verriegelungselemente zudem ein Verriegeln der formschlüssigen Verbindungen gegen unbeabsichtigtes Lösen. Im Gebrauch lässt sich der Steinfangkorb beim Auffalten und Zusammenziehen zwecks Einfangen eines Steins oder dergleichen in einem Körpergewebe problemlos in einer bestimmten axiale Lage halten, da nicht der mit dem Steinfangkorb verbundene Zugdraht, sondern der ihn umgebende Schlauch aktiv axial verschoben wird. Das gegenseitige elastische Vorspannen der beiden Anschlussstücke durch die Schraubenfeder oder ein anderes elastisches Element hält das Steinfangkorbinstrument selbsttätig in seiner den Steinfangkorb einziehenden Stellung. Dies ermöglicht zum einen das Einführen des Steinfangkorbinstruments mit vollständig im Schlauch zusammengefaltetem Drahtkorb ohne aktive Bedienbetätigung. Zum anderen hält das Steinfangkorbinstrument dadurch einen eingefangenen Stein selbsttätig klemmend fest, so dass auch in dieser Drahtkorbposition bei Bedarf die Griffeinheit vom Steinfangkorbinstrument demontiert werden kann, ohne dabei den Stein zu verlieren oder durch eine zusätzliche Bedienbetätigung das Festklemmen im Drahtkorb gewährleisten zu müssen.

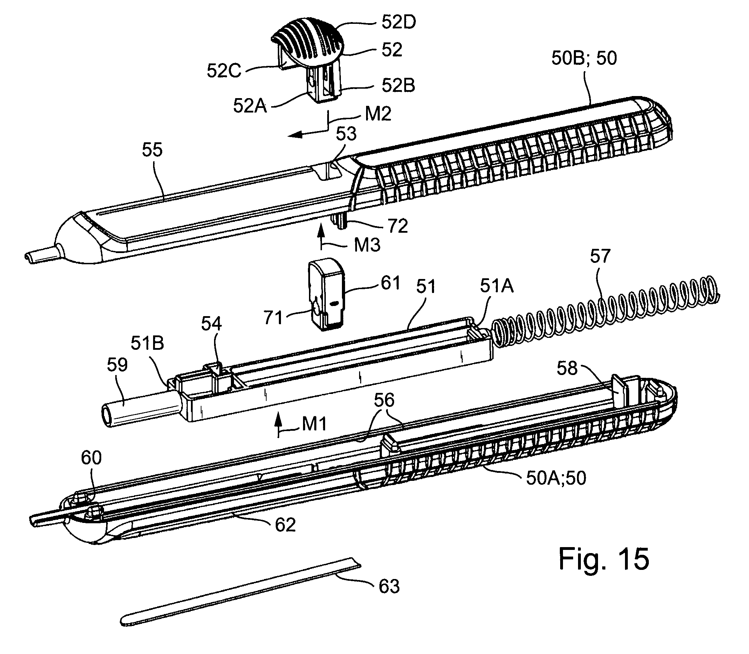

Die Figuren 15 bis 18C veranschaulichen eine Realisierung des erfindungsgemäßen Wechselgriffsystems. Diese Variante beinhaltet eine Griffeinheit, bei welcher ein erster Griffteil 50 einen Griffkörper aus einer unteren Griffschale 50A und einer auf diese aufklipsbaren oberen Griffschale 50B während ein zweiter Griffteil als ein aktiv vom Benutzer zu betätigendes, d.h. zu bewegendes Griffbedienteil in Form eines Schieberelementes ausgebildet ist, das durch eine am Griffkörper 50 vorgesehene Schlittenführung 56 axial beweglich an selbigem geführt ist.

Das Schieberelement umfasst einen Schlittenkörper 51 und einen Bedienkopfkörper 52, der querbeweglich am Schlittenkörper 51 gehalten ist. Dazu ist er mit einem Basisteil 52A durch eine korrespondierende Durchstecköffnung 53 an der Oberschale 50B hindurch in eine zugehörige Aufnahme 54 des Schlittenkörpers 51 eingesteckt und in diesem mittels seitlichen Klipsen 52B lösbar verrastet gehalten. Mit einem Stegteil 52C ist der Bedienkopfkörper 52 längs der Schlitzführung 55 in der Griffoberschale 50B beweglich geführt. Der Schlittenkörper 51 ist längs der an der Innenseite des Griffkörpers 50 gebildeten Schlittenführung 56 axial relativ zum Griffkörper 50 beweglich geführt.

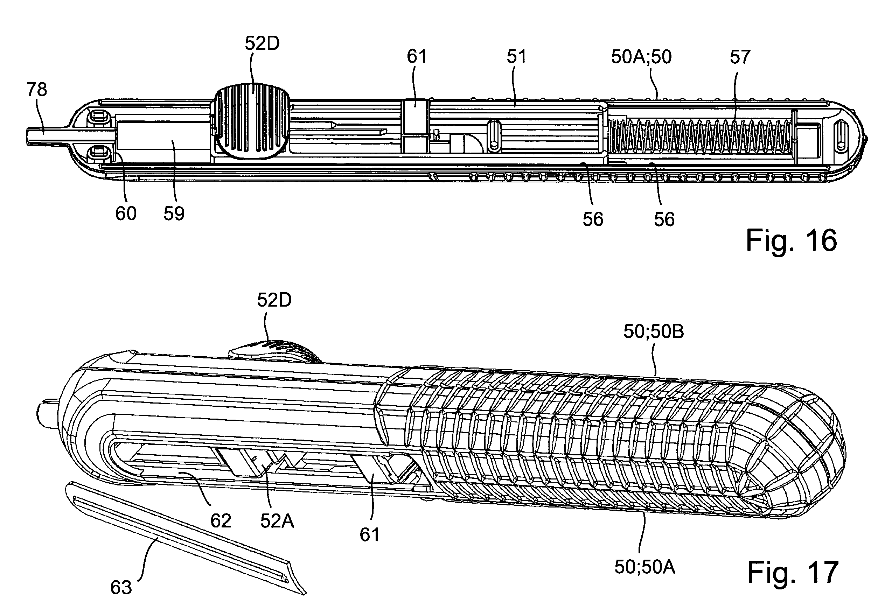

Eine Druckfeder 57 ist zwischen ein hinteres Ende 51A des Schlittenkörpers 51 und einen am Griffkörper 50 angeformten Anschlag 58 eingebracht und drückt den Schlittenkörper 51 in eine in Figur 16 gezeigte vordere Endstellung. Diese ist dadurch definiert, dass ein Hubbegrenzungsfortsatz 59 an einem vorderen Ende 51 B des Schlittenkörpers 51 gegen einen korrespondierenden vorderseitigen Gehäusekörperanschlag 60 zur Anlage kommt. Die axiale Länge des hubwegdefinierenden Fortsatzes 59 kann z.B. bei der Herstellung oder auch nachträglich variabel gewählt werden, so dass der Fortsatz 59 als variables Hubbegrenzungselement fungiert, mit dem der Hubweg der axialen Relativbeweglichkeit des Schlittenkörpers 51 relativ zum Griffkörper 50 entsprechend variabel einstellbar ist.

Mit einem vom Steg 52C getragenen Pilzkopfteil 52D ragt der Bedienkopfkörper 52 im fertig montierten Zustand aus dem Griffkörper 50 an der Oberseite der oberen Griffschale 50B heraus und fungiert als Kontaktelement, das der Benutzer beispielsweise mit einem Daumen kontaktiert, um die gewünschte Betätigung auszuführen. Außerdem beinhaltet die Griffeinheit einen im Griffkörper 50 zwischen einer sichernden und einer freigebenden Stellung querbeweglich in der Durchstecköffnung 53 gehaltenen Sicherungszapfen 61.

Zur Montage wird der Bedienkopfkörper 52 mit seinem Basisteil 52A durch die Durchstecköffnung 53 durchgesteckt und etwas nach vorn geschoben, so dass er an der Griffoberschale 50B verschieblich gehalten ist. Dann wird der Schlittenkörper 51 in die Oberschale 50B derart eingelegt, dass der Bedienkopfkörper 52 in seine Aufnahme 54 am Schlittenkörper 51 gelangt und in dieser festgeklipst wird. Diese Montageschritte sind mit einem Montagepfeil M2 in Figur 15 veranschaulicht. Anschließend wird der Sicherungszapfen 61 von unten her in die Durchstecköffnung 53 der Oberschale 50B eingefügt, wie mit einem Montagepfeil M3 veranschaulicht, und nach Einlegen der Druckfeder 57 wird die Unterschale 50A auf die Oberschale 50B geklipst, wie mit einem Montagepfeil M1 veranschaulicht. Ein an der Unterseite der Unterschale 50A vorgesehenes Schlitzfenster 62 wird durch lösbares Aufrasten einer Abdeckung 63 verschlossen.

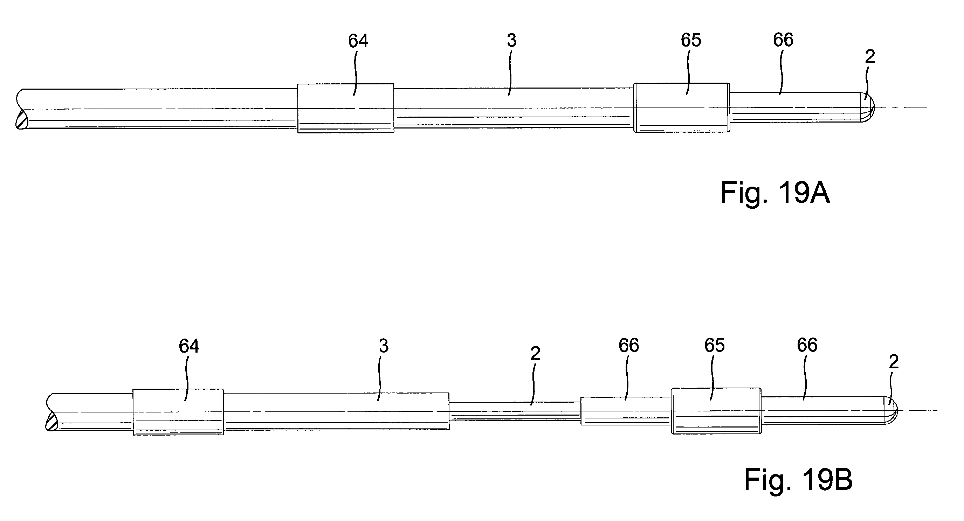

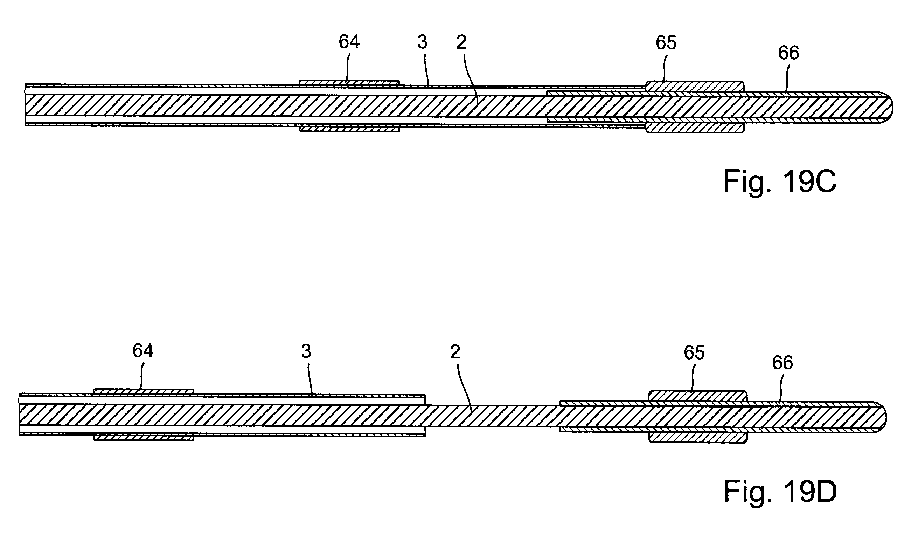

Auch diese Variante des Wechselgriffsystems eignet sich insbesondere zum einfach lösbaren Ankoppeln zweier langgestreckter, axial relativbeweglicher Funktionsteile einer entsprechenden medizinischen Instrumenteneinheit, wie eines Steinfangkorbinstruments. Speziell ist daran eine medizinische Instrumenteneinheit ankoppelbar, wie sie mit ihrem betreffenden Ankopplungsendbereich in den Figuren 19A bis 19D näher gezeigt ist. Wie im Fall der Figuren 1A bis 3C kann es sich insbesondere um ein Steinfangkorbinstrument 1 mit einem Zugdraht 2 und einem Schlauch 3 handeln, wobei zum einfacheren Verständnis für das Ausführungsbeispiel der Figuren 19A bis 19D gleiche Bezugszeichen für funktionell äquivalente Komponenten gewählt sind. Am Schlauch 3 ist kurz vor dessen hinterem Ende ein Haltering 64 bewegungsstarr fixiert, der den Schlauch 3 durchmesservergrößernd umgibt. In ähnlicher Weise ist am Zugdraht 2 kurz vor dessen hinterem Ende ein Haltering 65 bewegungsstarr fixiert, der den Zugdraht 2 durchmesservergrößernd umgibt. Dazu ist der Haltering 65 an einer Hülse 66 mit etwas größerer axialer Länge fixiert, die ihrerseits direkt am Zugdraht 2 fixiert ist. Die Durchmesser von Hülse 66 und Haltering 65 sind so gewählt, dass sich im zusammengeschobenen Zustand gemäß den Figuren 19A und 19C der Schlauch 3 mit seinem hinteren Ende auf die Hülse 66 aufschiebt und gegen den Haltering 65 zur Anlage kommt.

Die medizinische Instrumenteneinheit der Figuren 19A bis 19D ist mittels der Halteringe 64, 65, die auch als Wulste bezeichnet werden können, an das Wechselgriffsystem gemäß den Figuren 15 bis 18C ankoppelbar, wobei der Schlauch über seinen Haltering 64 axial bewegungsstarr mit dem Bedienelementkörper 52 und der Zugdraht 2 mit seinem Haltering 65 axial bewegungsstarr am Griffkörper 50 gehalten wird. Dazu sind insbesondere der Bedienelementkörper 52 und der Sicherungszapfen 61 geeignet gestaltet.

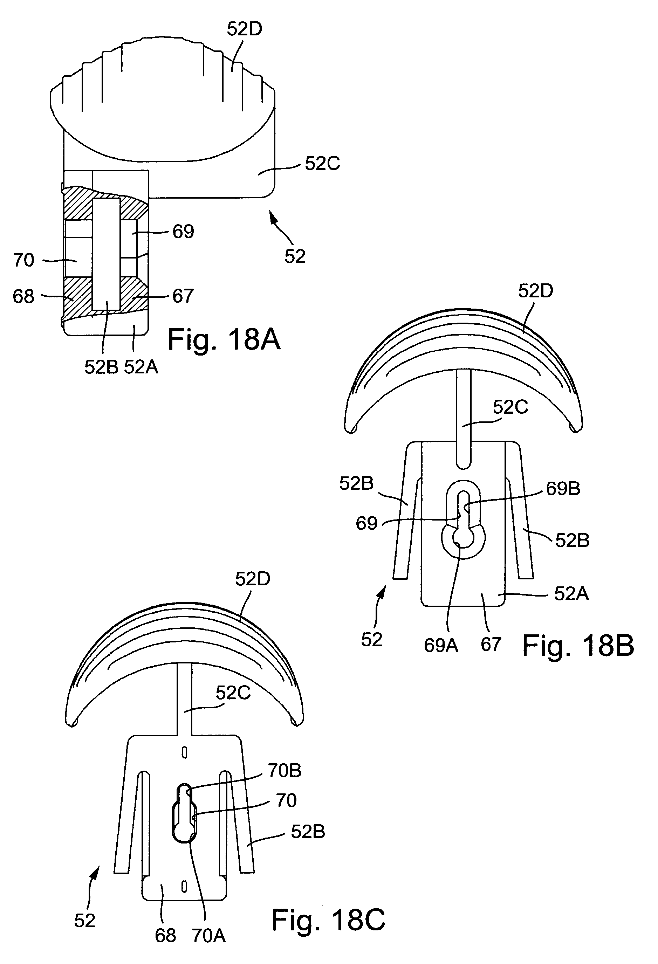

Speziell weist zu diesem Zweck der Basisteil 52A des Bedienelementkörpers 52 eine vordere Wandung 67 und eine davon axial beabstandete hintere Wandung 68 auf, in die je eine Schlitzöffnung 69, 70 eingebracht sind, wie in den Detailansichten der Figuren 18A bis 18C näher ersichtlich. Die Schlitzöffnungen 69, 70 weisen jeweils in einem zentralen Bereich 69a, 70a einen größeren Durchmesser als in einem außermittigen Abschnitt 69b, 70b auf. Dabei erstreckt sich der breitere Durchmesserbereich 69a, 70a in der Vorderwand 67 mit geringerer Querausdehnung als in der Rückwand 68. Korrespondierend dazu ist der Bedienkopfkörper 52 in seiner Aufnahme 54 derart eingeklipst, dass er zwischen einer Freigabestellung und einer verrastenden Stellung querbeweglich am Griffkörper 50 gehalten ist und zusätzlich eine Mittenstellung zwischen den beiden Endstellungen besitzt.

Bei angekoppelter medizinischer Instrumenteneinheit ist der Schlauch 3 axial bewegungsstarr am Bedienelementkörper 52 gehalten, indem sein Haltering 64 zwischen Vorderwand 67 und Rückwand 68 im Bereich der engeren Schlitzöffnungsabschnitte 69b, 70b festgehalten wird. Zum entsprechenden Halten des Zugdrahts 2 am Griffkörper 50 weist der Sicherungszapfen 61 eine ähnliche Schlitzöffnung 71 mit einem breiteren mittigen Abschnitt und einem verengten außermittigen Abschnitt auf. Wie bei den Schlitzöffnungen 69, 70 des Bedienelementkörpers 52 ist auch am Sicherungszapfen 61 der Öffnungsdurchmesser so gewählt, dass der betreffende Haltering 64 bzw. 65 durch den größeren Durchmesserbereich hindurchtreten kann, vom verengten Durchmesserbereich hingegegen zurückgehalten wird, d.h. der Durchmesser des jeweiligen Halterings 64, 65 ist kleiner als der Öffnungsdurchmesser im mittigen, breiteren Bereich 69A, 70A und kleiner als im verengten Schlitzöffnungsbereich 69B, 70B am Bedienelementkörper 50 bzw. an der Schlitzöffnung 71 des Sicherungszapfens 61.

Wie der Bedienelementkörper 52 ist auch der Sicherungszapfen 61 querbeweglich am Griffkörper 50 gehalten, so dass in einer Freigabestellung sein breiterer Schlitzöffnungsbereich in der Längsachse der Griffeinheit liegt, längs der sich die eingelegte medizinische Instrumenteneinheit 1 erstreckt, während er in einer sichernden Verriegelungsstellung den Haltering 65 und damit den Zugdraht 2 durch seinen engeren Schlitzabschnitt zurückhält. In dieser letztgenannten Gebrauchsstellung liegt der Zugdraht 2 mit seinem hinteren Endbereich quasi spielfrei zwischen dem Sicherungszapfen 61, der ihn an seinem Haltering 65 zurückhält, und einem dahinterliegenden Endanschlag 72 des Gehäusekörpers.

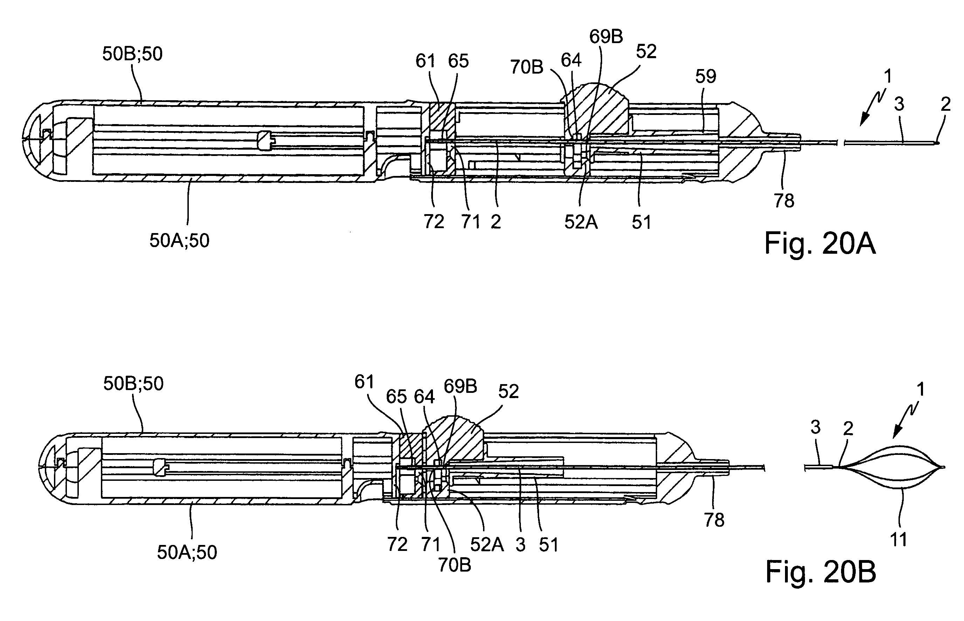

Die Figuren 20A und 20B veranschaulichen diese vorteilhafte Ankopplung der medizinischen Funktionseinheit 1 mit Zugdraht 2 und Schlauch 3 an das Wechselgriffsystem gemäß Figur 15 am Beispiel eines Steinfangkorbinstruments.

Zur Montage der Instrumenteneinheit 1 wird diese mit ihrem hinteren Endbereich in ein vorderseitiges Mundstück 78 des Griffkörpers 50 eingeschoben, bis der Schlauchhaltering 64 die Schlitzöffnung 69 in der Vorderwand 67 des Bedienelementkörpers 52 passiert hat, der sich dazu in seiner Freigabestellung befindet. Dann wird der Bedienelementkörper 52 in seine Mittenstellung verbracht und gegen die Kraft der Druckfeder 57 nach hinten bewegt, wobei er in dieser Mittenstellung den Haltering 64 durch den engeren Schlitzabschnitt 69B der Vorderwand 67 mitnimmt und dabei die gesamte Instrumenteneinheit nach hinten schiebt, bis der Bedienelementkörper 52 seine hintere Endstellung erreicht hat. In dieser Endstellung kommt der Zugdraht 2 gegen den Endanschlag 72 am Gehäusekörper 50 zur Anlage, und der Haltering 65 hat die Schlitzöffnung 71 des in seiner Freigabestellung befindlichen Sicherungszapfens 61 passiert und befindet sich direkt hinter dieser. Daraufhin wird der Sicherungszapfen 61 quer in seine verrastende, sichernde Stellung bewegt, und ebenso wird der Bedienelementkörper 52 quer in seine verrastende, sichernde Stellung bewegt, in welcher der Schlauchhaltering 64 zwischen den engen Schlitzabschnitten 69B, 70B der beiden Wandungen 67, 68 des Stegteils 52A des Bedienelementkörpers 52 eingeklemmt gehalten ist. Von Vorteil ist dabei, dass in dieser axialen Position von Instrumenteneinheit und Bedienelementkörper 52 der Schlauchhaltering 64 gerade zwischen den beiden Schlitzöffnungen 69, 70 des Bedienelementkörpers 52 liegt und damit durch die Querbewegung des Bedienelementkörpers 52 automatisch in seine festgeklemmte Stellung gelangt. Der Montagevorgang ist damit beendet, und der Bedienelementkörper 52 kann wieder in seine vordere Ausgangsstellung bewegt werden, in welcher ihn die Druckfeder 57 gedrückt hält.

Wie die obige Erläuterung deutlich macht, ermöglicht das Wechselgriffsystem eine sehr einfache Montage der Drahtinstrumenteneinheit, ohne dass dazu der Griffkörper 50 auseinander gebaut werden muss. In gleicher Weise ist eine einfache Demontage ohne Auseinanderbauen des Griffkörpers 50 möglich, wozu nach Öffnen der Abdeckung 63 der Sicherungszapfen 61 und der Bedienelementkörper 52 von unten her in ihre Freigabestellung querbewegt werden. Die Instrumenteneinheit 1 kann dann einfach nach vorne aus dem Griffkörper herausgezogen werden.

Figur 20A zeigt das fertig montierte Steinfangkorbinstrument in seiner Ausgangsstellung, in welcher sich der Bedienelementkörper 52 in seiner vorderen Endstellung befindet und das am distalen Ende des Zugdrahts 2 vorgesehene Drahtkörbchen 11 ganz in den Schlauch 3 eingezogen ist. Figur 20B zeigt das Instrument in der Stellung, in welcher der Bedienelementkörper 52 in seine hintere Endstellung zurückgedrückt ist. Bei dieser Rückbewegung nimmt der Bedienelementkörper 52 den angekoppelten Schlauch 3 mit, während der Zugdraht 2 stationär bleibt. Dies hat zur Folge, dass sich am distalen Endbereich der Schlauch 3 zurückzieht und das Drahtkörbchen 11 in seinen entfalteten Zustand freigibt.

Im übrigen weist das Instrument gemäß den Fig. 15 bis 20B analoge Eigenschaften und Vorteile auf, wie sie oben zum Instrument der Fig 1A bis 14 angegeben sind, worauf verwiesen werden kann.

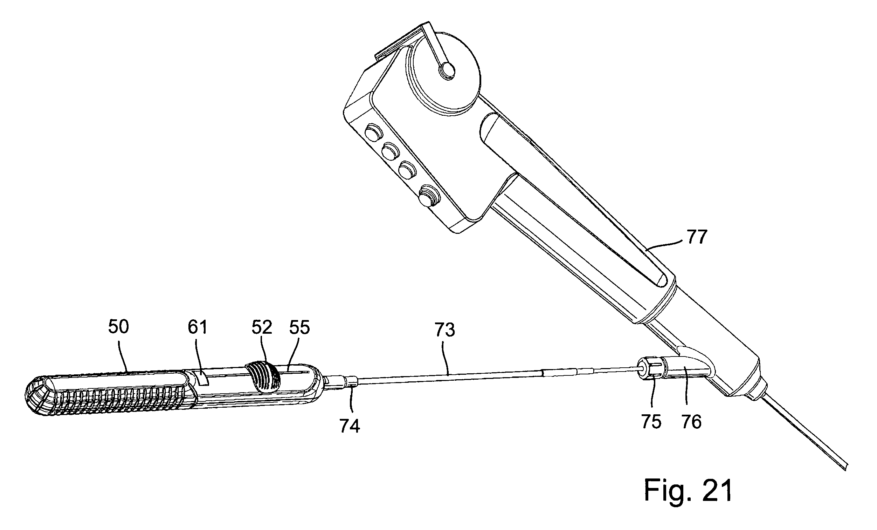

Figur 21 zeigt eine vorteilhafte Ankopplungsmöglichkeit des Steinfangkorbinstruments der Figuren 20A und 20B an ein Endoskop. Dazu ist eine Teleskop-Adapterhülse 73 vorgesehen, die am einen Ende mit einem zum Gehäusekörper-Mundstück 80 passenden Anschluss 74 und am anderen Ende mit einem Luer-Anschluss 75 versehen ist, mit dem sie an einen entsprechenden Anschluss 76 eines Endoskopgriffteils 77 herkömmlicher Art angekoppelt werden kann. Die Adapterhülse 73 ist in ihrer Länge teleskopartig variabel verstellbar.

Es versteht sich, dass das erfindungsgemäße Wechselgriffsystem außer in den gezeigten in vielen weiteren Ausführungsformen realisierbar ist, mit denen sich die oben zu den gezeigten Beispielen erwähnten Vorteile je nach Systemrealisierung ganz oder jedenfalls teilweise erzielen lassen. Insbesondere ist das erfindungsgemäße Wechselgriffsystem selbstverständlich auch für andere medizinische Instrumente verwendbar, die zwei langgestreckte, axial relativbewegliche Funktionsteile aufweisen, welche lösbar an ein Griffsystem anzukoppeln sind, wie beispielsweise medizinische Instrumente, die wie das geschilderte Steinfangkorbinstrument einen Zugdraht und einen diesen umgebenden, relativ zu diesem axial beweglichen Schlauch aufweisen, wobei statt des Steinfangkorbs ein anderes Funktionselement am distalen Instrumentende angeordnet ist, z.B. ein Schneidelement, ein Filterelement etc. Auch sind alternativ zu den gezeigten, scheibenförmigen bzw. schieber- oder zapfenartigen Verriegelungselementen je nach Systemauslegung andere Verriegelungselemente herkömmlicher Art verwendbar, z.B. solche, die auf einem Klappmechanismus beruhen. Wesentlich ist insoweit allein die gegen unbeabsichtigtes Lösen der axial bewegungsstarren Koppelverbindung von Zugdraht bzw. Schlauch mit dem betreffenden Griffteil sichernde Verriegelungsfunktion. Weiter versteht sich, dass es sich bei den beiden langgestreckten, axial relativbeweglichen Funktionsteilen eines an das erfindungsgemäße Wechselgriffsystem lösbar ankoppelbaren medizinischen Instruments nicht zwingend um einen langgestreckten Draht und einen diesen umgebenden Schlauch handeln muss, wie gezeigt, sondern auch um zwei andere Funktionsteile, je nach dem medizinischen Instrumententyp, z.B. um zwei axial relativbewegliche Funktionsdrähte, die nebeneinander oder koaxial angeordnet sind. Weiter versteht sich, dass das erfindungsgemäße Wechselgriffsystem nicht auf das Anwendungsgebiet medizinischer Instrumente beschränkt ist, sondern in gleicher Weise auf anderen Gebieten anwendbar ist, bei denen der Bedarf besteht, zwei langgestreckte, axial relativbewegliche Funktionsteile lösbar an eine Bediengriffeinheit ankoppeln zu können.

## Patentansprüche

1. Wechselgriffsystem zum lösbaren Ankoppeln zweier langgestreckter, axial relativbeweglicher Funktionsteile (2, 3), mit
- einem ersten und einem zweiten Griffteil (50, 51, 52), die axial relativbeweglich angeordnet sind,
- einem ersten Kopplungsmittel (61) zum lösbaren, axial bewegungsstarren Koppeln eines ersten (2) der beiden Funktionsteile mit dem ersten Griffteil (50) und
- einem zweiten Kopplungsmittel (52A) zum lösbaren, axial bewegungsstarren Koppeln des zweiten (3) der beiden Funktionsteile mit dem zweiten Griffteil (51, 52),
- wobei der zweite Griffteil (51, 52) eine Durchführungsöffnung (69, 70) zum Durchführen wenigstens des ersten (2) der beiden Funktionsteile aufweist **dadurch gekennzeichnet dass**,
- der erste Griffteil (50) einen Griffkörper aus einer unteren Griffschale (50A) und einer auf diese aufklipsbaren oberen Griffschale (50B) beinhaltet und der zweite Griffteil ein an der Oberseite der oberen Griffschale (50B) herausragendes, daumenbetätigtes Pilzkopfteil (52D) aufweist.

2. Wechselgriffsystem nach Anspruch 1, weiter **dadurch gekennzeichnet, dass** das erste und/oder das zweite Kopplungsmittel ein zugeordnetes betätigbares Sicherungsmittel (52A, 61) zum Sichern der zugehörigen Koppelverbindung von Funktionsteil und Griffteil aufweist.

3. Wechselgriffsystem nach Anspruch 1 oder 2, weiter **dadurch gekennzeichnet, dass**
- das erste Kopplungsmittel ein erstes Koppelelement (61) am ersten Griffteil aufweist, mit dem ein erstes Gegenkoppelelement (65) an einem hinteren Endbereich des ersten Funktionsteils lösbar koppelbar ist, und/oder
- das zweite Kopplungsmittel ein zweites Koppelelement (52A) aufweist, mit dem ein zweites Gegenkoppelelement (64) an einem hinteren Endbereich des zweiten Funktionsteils lösbar koppelbar ist.

4. Wechselgriffsystem nach Anspruch 3, weiter **dadurch gekennzeichnet, dass** das erste Koppelelement und/oder das zweite Koppelelement eine jeweilige Nut oder Nutaufnahme (23, 26) zur Bildung eines formschlüssigen Koppelelement-Gegenkoppelelement-Paares mit einer jeweils korrespondierenden Nutaufnahme oder Nut als Gegenkoppelelement oder eine jeweilige Schlitzöffnung (69, 70, 71) zur Bildung eines formschlüssigen Paares mit einem jeweiligen Haltering- oder Wulstelement (64, 65) beinhaltet.

5. Wechselgriffsystem nach Anspruch 4, weiter **dadurch gekennzeichnet, dass** das Sicherungsmittel für das jeweilige Nut-Nutaufnahme-Paar ein betätigbares Verriegelungselement (52, 61) beinhaltet, das die Nut-Nutaufnahme-Verbindung gegen Lösen verriegelt und zum Lösen entriegelt.

6. Wechselgriffsystem nach einem der Ansprüche 1 bis 5, weiter **gekennzeichnet durch** ein variables Hubbegrenzungselement (59) zum variablen Einstellen eines Hubweges der axialen Relativbeweglichkeit von erstem und zweitem Griffteil.

7. Wechselgriffsystem nach einem der Ansprüche 1 bis 6, weiter **gekennzeichnet durch** ein elastisches Vorspannelement (57), das in Axialrichtung zwischen den beiden Griffteilen wirkt.

8. Wechselgriffsystem nach einem der Ansprüche 1 bis 7, weiter **dadurch gekennzeichnet, dass**
- sich die Durchführungsöffnung in einem vorderen Endbereich des vorderen Griffteils trichterförmig nach außen weitet und/oder
- der Griffkörper (50) des ersten Griffteils eine Schlittenführung (56) aufweist und der zweite Griffteil einen aktiv zu betätigenden Griffbedienteil (51, 52) beinhaltet, der als Schieberelement (51, 52) ausgebildet ist, das durch die Schlittenführung axial beweglich am Griffkörper geführt ist.

9. Wechselgriffsystem nach Anspruch 8, weiter **dadurch gekennzeichnet, dass** das Schieberelement einen Schlittenkörper (51) und einen am Schlittenkörper querbeweglich gehaltenen Bedienelementkörper (52) beinhaltet, der das zugeordnete Sicherungsmittel zum Sichern der Koppelverbindung von zweitem Griffteil und zweitem Funktionsteil bildet, wobei er zwischen einer verrastenden Schiebebedienstellung und einer entsichernden Stellung querbeweglich ist.

10. Medizinisches Instrument mit
- einem Wechselgriffsystem nach einem der Ansprüche 1 bis 9 und
- einer medizinischen Instrumenteneinheit mit
- einem ersten langgestreckten Funktionsteil (2) mit einem an einem hinteren Endbereich vorgesehenen ersten Gegenkoppelelement (4a, 6; 65) zum lösbaren, axialen bewegungsstarren Koppeln mit einem ersten Griffteil eines Wechselgriffsystems und
- einem gegenüber dem ersten axial relativbeweglichen, zweiten langgestreckten Funktionsteil (3) mit einem an einem hinteren Endbereich vorgesehenen zweiten Gegenkoppelelement (4b, 10; 64) zum lösbaren, axial bewegungsstarren Koppeln mit einem zweiten Griffteil des Wechselgriffsystems,
- wobei das erste und das zweite Gegenkoppelelement (4a, 4b, 6, 10; 64, 65) eine Anschlusseinheit mit so geringem Außendurchmesser bilden, dass ein zugeordneter Katheter-/Endoskopieschlauch über selbige hinwegziehbar ist.

11. Medizinisches Instrument nach Anspruch 10, weiter **dadurch gekennzeichnet, dass** das erste Gegenkoppelelement ein am ersten Funktionsteil vorgesehenes erstes Anschlussstück (4a) und das zweite Gegenkoppelelement ein am zweiten Funktionsteil vorgesehenes zweites Anschlussstück (4b) beinhaltet und ein elastisches Element (9) vorgesehen ist, das in Axialrichtung zwischen den beiden Anschlussstücken wirkt.

12. Medizinisches Instrument nach Anspruch 11, weiter **dadurch gekennzeichnet, dass** die beiden Funktionsteile ein Zugdraht (2) und ein diesen umgebender Schlauch (3) sind.

13. Medizinisches Instrument nach Anspruch 12, weiter **dadurch gekennzeichnet, dass**
- der Zugdraht das erste Funktionsteil und der Schlauch das zweite Funktionsteil bildet und/oder
- die medizinische Instrumenteneinheit eine Steinfangkorbinstrumenteneinheit (1) ist.

14. Medizinisches Instrument nach Anspruch 12 oder 13, weiter **gekennzeichnet durch** eine den Schlauch umgebende Einführhilfshülse (41), die in einer Ruheposition lösbar am vorderen Griffteil angebracht ist und aus der Ruheposition nach vorn bis zum distalen Endbereich von Zugdraht und Schlauch in eine Einführhilfsposition bewegbar ist.

15. Medizinisches Instrument nach einem der Ansprüche 10 bis 14, weiter **dadurch gekennzeichnet, dass** es ein Endoskopieinstrument mit einem Endoskopiegriffkörper (77) ist, wobei das Endoskopieinstrument eine Teleskop-Adapterhülse (73) zur Ankopplung der medizinischen Instrumenteneinheit an den Endoskopiegriffkörper aufweist.

## Claims

1. A change-out handle system for releasably coupling two elongated, axially relatively displaceable functional parts (2, 3), comprising
- a first and a second handle part (50, 51, 52), which are arranged so as to be axially relatively displaceable,
- a first coupling means (61) for the releasable, axially rigid coupling of a first (2) of the two functional parts to the first handle part (50) and
- a second coupling means (52A) for the releasable, axially rigid coupling of the second (3) of the two functional parts to the second handle part (51, 52),
- wherein the second handle part (51, 52) has an opening (22; 69, 70) for passing through at least the first (2) of the two functional parts, **characterized in that**
- the first handle part (50) comprises a handle body formed by a lower handle shell (50A) and an upper handle shell (50B) to be clipped thereon, and the second handle part comprises a mushroom head part (52D) protruding out of the upper handle shell (50B) at a top side thereof for being operated by a thumb.

2. The change-out handle system as claimed in claim 1, further **characterized in that** the first and/or the second coupling means has an associated manipulatable securing means (52A, 61) for securing the associated coupling connection between functional part and handle part.

3. The change-out handle system as claimed in claim 1 or 2, further **characterized in that**
- the first coupling means has a first coupling element (61) on the first handle part, by way of which a first counter coupling element (65) is releasably couplable to a rear end region of the first functional part, and/or
- the second coupling means has a second coupling element (52A), by way of which a second counter coupling element (64) is releasably couplable to a rear end region of the second functional part.

4. The change-out handle system as claimed in claim 3, further **characterized in that** the first coupling element and/or the second coupling element includes a respective groove or groove receiving means (23, 26) for forming a positive-locking coupling element/counter coupling element pair with a respectively corresponding groove receiving means or groove as counter coupling element or a respective slot opening (69, 70, 71) for forming a positive-locking pair with a respective retaining ring element or bead element (64, 65).

5. The change-out handle system as claimed in claim 4, further **characterized in that** the securing means for the respective groove/groove receiving means pair includes a manipulatable locking element (52, 61), which locks the groove/groove receiving means connection against release and unlocks it for release.

6. The change-out handle system as claimed in any one of claims 1 to 5, further **characterized by** a variable stroke limiting element (59) for variable adjustment of a stroke path of axial relative displaceability of the first and second handle part.

7. The change-out handle system as claimed in any one of claims 1 to 6, further **characterized by** a resilient prestressing element (57) which acts in the axial direction between the two handle parts.

8. The change-out handle system as claimed in any one of claims 1 to 7, further **characterized in that**
- the opening widens outwards in the shape of a funnel in a front end region of the front handle part, and/or
- the handle body (50) of the first handle part comprises a carriage guide (56), and the second handle part includes a handle control part (51, 52) which is to be actively manipulated and is realized as a slider element (51, 52) which is guided on the handle body in an axially displaceable manner by the carriage guide.

9. The change-out handle system as claimed in claim 8, further **characterized in that** the slider element includes a carriage body (51) and a control element body (52), which is held so as to be transversely movable on the carriage body and forms the associated securing means for securing the coupling connection between the second handle part and the second functional part, wherein it is transversely displaceable between a latching slide control position and a position disengaging the securing function.

10. A medical instrument comprising
- a change-out handle system as claimed in any one of claims 1 to 9 and
- a medical instrument unit, having
- a first elongated functional part (2) with a first counter coupling element (4a, 6; 65) provided on a rear end region for the releasable, axial rigid coupling to a first handle part of a change-out handle system and
- a second elongated functional part (3), which is axially relatively displaceable with respect to the first functional part and comprises a second counter coupling element (4b, 10; 64) provided on a rear end region for the releasable, axially rigid coupling to a second handle part of the change-out handle system,
- wherein the first and the second counter coupling element (4a, 4b, 6, 10; 64, 65) form a connection unit having an outside diameter small enough to allow a corresponding catheterendoscopy tube to be pulled over the same.

11. The medical instrument as claimed in claim 10, further **characterized in that** the first counter coupling element includes a first connection piece (4a) provided on the first functional part and the second counter coupling element includes a second connection piece (4b) provided on the second functional part, and **in that** a resilient element (9) is provided which acts in axial direction between the two connection pieces.

12. The medical instrument as claimed in claim 11, further **characterized in that** the two functional parts are a wire pull (2) and a tube (3) surrounding said wire pull.

13. The medical instrument as claimed in claim 12, further **characterized in that**
- the wire pull forms the first functional part and the tube forms the second functional part, and/or
- the medical instrument unit is a stone catching instrument unit (1).

14. The medical instrument unit as claimed in claim 12 or 13, further **characterized by** an entry-aiding sleeve (41), which surrounds the tube and in a rest position is attached releasably on the front handle part and is displaceable out of the rest position forward as far as the distal end region of the wire pull and tube to an entry-aiding position.

15. The medical instrument as claimed in any one of claims 10 to 14, further **characterized in that** it is an endoscopy instrument with an endoscopy handle body (77), wherein the endoscopy instrument has a telescopic adapter sleeve (73) for coupling the medical instrument unit to the endoscopy handle body.

## Revendications

1. Système de préhension interchangeable pour accoupler de manière détachable deux pièces fonctionnelles (2, 3) allongées relativement mobiles axialement, comportant
- une première et une deuxième partie de préhension (50, 51, 52) qui sont agencées de manière relativement mobile axialement,
- un premier moyen d'accouplement (61) pour accoupler de manière détachable et relativement rigide axialement une première (2) des deux pièces fonctionnelles avec la première partie de préhension (50), et
- un deuxième moyen d'accouplement (52A) pour accoupler de manière détachable et relativement rigide axialement la deuxième (3) des deux pièces fonctionnelles avec la deuxième partie de préhension (51, 52),
- dans lequel la deuxième partie de préhension (51, 52) présente un orifice de passage (69, 70) pour faire passer au moins la première (2) des deux pièces fonctionnelles, **caractérisé en ce que**
la première partie de préhension (50) contient un corps de préhension constitué par une coque de préhension inférieure (50A) et par une coque de préhension supérieure (50B) susceptible d'être enclipsée sur celle-ci, et la deuxième partie de préhension présente une partie de tête en forme de champignon (52D) actionnée au moyen du pouce et dépassant au-delà de la face supérieure de la coque de préhension (50B) supérieure.

2. Système de préhension interchangeable selon la revendication 1, **caractérisé en outre en ce que** le premier et/ou le deuxième moyen d'accouplement présente un moyen de sécurité (52A, 61) actionnable associé pour assurer la liaison par accouplement associée de la pièce fonctionnelle et de la partie de préhension.

3. Système de préhension interchangeable selon la revendication 1 ou 2, **caractérisé en outre en ce que**
- le premier moyen d'accouplement présente un premier élément d'accouplement (61) sur la première partie de préhension, avec lequel un premier élément d'accouplement antagoniste (65) peut être accouplé de manière détachable sur une zone d'extrémité postérieure de la première pièce fonctionnelle, et/ou
- le deuxième moyen d'accouplement présente un deuxième élément d'accouplement (52A) avec lequel un deuxième élément d'accouplement antagoniste (64) peut être accouplé de manière détachable sur une zone d'extrémité postérieure de la deuxième pièce fonctionnelle.

4. Système de préhension interchangeable selon la revendication 3, **caractérisé en outre en ce que** le premier élément d'accouplement et/ou le deuxième élément d'accouplement contient une rainure ou un logement de rainure (23, 26) pour former une paire élément d'accouplement/élément d'accouplement antagoniste par coopération de formes avec un logement de rainure ou une rainure respectivement correspondant(e) ou une ouverture en forme de fente (69, 70, 71) respective pour former une paire par coopération de formes avec un élément respectif de bague de retenue ou de bourrelet (64, 65).

5. Système de préhension interchangeable selon la revendication 4, **caractérisé en outre en ce que** le moyen de sécurité pour la paire respective rainure/logement de rainure contient un élément de verrouillage (52, 61) actionnable qui verrouille la liaison rainure/logement de rainure à l'encontre d'un détachement et qui la déverrouille pour la détacher.

6. Système de préhension interchangeable selon l'une des revendications 1 à 5, **caractérisé en outre par** un élément de limitation de course (59) variable pour le réglage variable d'une course de levage de la mobilité relative axiale de la première et de la deuxième partie de préhension.

7. Système de préhension interchangeable selon l'une des revendications 1 à 6, **caractérisé en outre par** un élément de précontrainte (57) élastique qui agit en direction axiale entre les deux parties de préhension.

8. Système de préhension interchangeable selon l'une des revendications 1 à 7, **caractérisé en outre en ce que**
- l'orifice de passage s'évase en forme d'entonnoir vers l'extérieur dans une zone d'extrémité antérieure de la partie de préhension antérieure,
- le corps de préhension (50) de la première partie de préhension présente un guide de chariot (56) et la deuxième partie de préhension contient une pièce de commande de préhension (51, 52) à actionner de manière active, qui est réalisée sous forme de poussoir (51, 52) qui est guidé par le guide de chariot de manière axialement mobile sur le corps de préhension.

9. Système de préhension interchangeable selon la revendication 8, **caractérisé en outre en ce que** l'élément poussoir contient un corps de chariot (51) et un corps d'élément de commande (52) maintenu transversalement mobile sur le corps de chariot, qui forme le moyen de sécurité associé pour assurer la liaison d'accouplement de la deuxième partie de préhension et de la deuxième pièce fonctionnelle, celui-ci étant mobile transversalement entre une position de commande à poussoir à encliquetage et une position débloquée.

10. Instrument médical comportant
- un système de préhension interchangeable selon l'une des revendications 1 à 9 et
- une unité d'instrument médical comportant
- une première pièce fonctionnelle (2) allongée avec un premier élément d'accouplement antagoniste (4a, 6 ; 65) prévu sur une zone d'extrémité postérieure pour effectuer l'accouplement rigide au mouvement axial de manière détachable avec une première partie de préhension d'un système de préhension interchangeable, et
- une deuxième pièce fonctionnelle (3) allongée relativement mobile axialement par rapport à la première, comportant un deuxième élément d'accouplement antagoniste (4b, 10 ; 64) prévu sur une zone d'extrémité postérieure pour effectuer l'accouplement rigide au mouvement axial de manière détachable avec une deuxième partie de préhension d'un système de préhension interchangeable,
- dans laquelle le premier et le deuxième élément d'accouplement antagoniste (4a, 4b, 6, 10 ; 64, 65) forment une unité de raccordement avec un diamètre extérieur aussi réduit qu'un tuyau de cathéter/d'endoscopie associé peut être tiré au moyen de celle-ci.

11. Instrument médical selon la revendication 10, **caractérisé en outre en ce que** le premier élément d'accouplement antagoniste contient une première pièce de raccordement (4a) prévue sur la première pièce fonctionnelle et le deuxième élément d'accouplement antagoniste contient une deuxième pièce de raccordement (4b) prévue sur la deuxième pièce fonctionnelle et **en ce qu'**il est prévu un élément (9) élastique qui agit en direction axiale entre les deux pièces de raccordement.

12. Instrument médical selon la revendication 11, **caractérisé en outre en ce que** les deux pièces fonctionnelles sont un fil de traction (2) et une gaine (3) entourant celui-ci.

13. Instrument médical selon la revendication 12, **caractérisé en outre en ce que**
- le fil de traction forme la première pièce fonctionnelle et la gaine la deuxième pièce fonctionnelle et/ou
- l'unité d'instrument médical est une unité d'instrument à panier extracteur de calculs (1).

14. Instrument médical selon la revendication 12 ou 13, **caractérisé en outre par** une douille facilitant l'introduction (41) entourant la gaine, qui dans une position de repos, est montée de manière détachable sur la partie de préhension antérieure et qui, depuis la position de repos, peut être déplacée vers l'avant jusqu'à la zone d'extrémité distale du fil de traction et de la gaine dans une position facilitant l'introduction.

15. Instrument médical selon l'une des revendications 10 à 14, **caractérisé en outre en ce qu'**il s'agit d'un instrument endoscopique avec un corps de préhension endoscopique (77), l'instrument endoscopique présentant une douille adaptatrice télescopique (73) pour accoupler l'unité d'instrument médical au corps de préhension endoscopique.
